# EUROPEAN PATENT APPLICATION

(11) **EP 4 807 005 A1**
(43) Date of publication of application: **16.09.2026**
(21) Application number: 24888685.5
(22) Date of filing: 05.11.2024
(51) Int. Cl.: C12N 15/62, C12N 15/12, C12N 15/13, C12N 15/54, C12Q 1/6897, C12Q 3/00, G01N 33/53, G01N 33/531

(54) **TARGET-MOLECULE-SPECIFIC FUNCTIONAL PROTEIN CONTROL SYSTEM**

(30) Priority: 06.11.2023 JP 2023189563
(71) Applicant: Kyoto University, Kyoto-shi, Kyoto 606-8501 (JP)
(72) Inventor: SAITO, Hirohide, Kyoto-shi, Kyoto 606-8501 (JP); KOMATSU, Shodai, Kyoto-shi, Kyoto 606-8501 (JP)
(74) Representative: HGF
(86) International application number: PCT/JP2024/039227
(87) International publication number: WO 2025/100390

(57) **Abstract**

A target molecule-specific functional protein regulation system including: the following (a) and (b): (a) a first nucleic acid molecule including a nucleic acid sequence encoding a first fusion protein that includes a first target binding domain including a light-chain variable domain of an antibody that specifically recognizes a target molecule, and a first domain of a functional protein; and (b) a second nucleic acid molecule including a nucleic acid sequence encoding a second fusion protein that includes a second target binding domain including a heavy-chain variable domain of an antibody that specifically recognizes the target molecule, and a second domain of a functional protein; or the following (c) and (d): (c) the first fusion protein; and (d) the second fusion protein, wherein a framework region of either the light-chain variable domain or the heavy-chain variable domain includes a sequence of a framework region of trastuzumab or a mutated sequence thereof, and the target molecule, the first target binding domain, and the second target binding domain form a ternary complex.

## Description

### TECHNICAL FIELD

The present invention relates to a target-molecule-specific functional protein regulation system and to a method therefor.

### BACKGROUND ART

Regulating gene expression in response to specific intracellular molecules is a powerful strategy for monitoring cellular conditions and regulating cellular programs. In nature, metabolic systems use transcription and translation regulators to monitor the consumption of cellular metabolites and precisely regulate metabolic pathways.

Artificial molecular systems capable of regulating gene expression in response to intracellular molecules in such a manner have been developed. A technique is known that regulates split RNA polymerases by the addition of exogenous compounds (see, for example, Patent Document 1). Techniques are also known that use two antibodies binding to a target molecule to achieve transcription or translation in response to an intracellular molecule (for example, Non-Patent Documents 1 to 3). Techniques are known that perform genome editing in response to intracellular miRNAs (for example, Non-Patent Document 4) as well as in response to extracellular molecules (for example, Non-Patent Document 5).

### REFERENCE DOCUMENT LIST

### PATENT DOCUMENT

| | |
|---|---|
| Patent Document 1: | WO 2017/212400 A2 |

### NON-PATENT DOCUMENTS

Non-Patent Document 1: Cao, J., Zhong, N., Wang, G., et al. Nanobody-based sandwich reporter system for living cell sensing influenza A virus infection. Sci Rep 9, 15899 (2019)
Non-Patent Document 2: Hideyuki Nakanishi, Hirohide Saito, and Keiji Itaka, Versatile Design of Intracellular Protein-Responsive Translational Regulation System for Synthetic mRNA. ACS Synthetic Biology 11 (3), 1077-1085 (2022)
Non-Patent Document 3: Cella, F., Wroblewska, L., Weiss, R., et al. Engineering protein-protein devices for multilayered regulation of mRNA translation using orthogonal proteases in mammalian cells. Nat. Commun. 9, 4392 (2018)
Non-Patent Document 4: Moe Hirosawa, et al. , Cell-type-specific genome editing with a microRNA-responsive CRISPR-Cas9 switch, Nucleic Acids Research, Volume 45, Issue 13, 27 July 2017, Page e118
Non-Patent Document 5: Toni A. Baeumler et al., Engineering Synthetic Signaling Pathways with Programmable dCas9-Based Chimeric Receptors. Cell Report, 20, 2639-2653, 2017

### SUMMARY OF THE INVENTION

### PROBLEM TO BE SOLVED BY THE INVENTION

However, none of the systems were sufficient as systems capable of responding to any molecule within the cell. That is, Patent Document 1 fails to achieve regulation of the molecular-dependent split RNA polymerase present within the cell. Although Non-Patent Documents 1 to 3 have successfully achieved transcription or translation in response to intracellular molecules, they all require two antibodies that bind to a target molecule. When using the two antibodies that bind to a target molecule, it is necessary to design them to bind to different sites on the target molecule surface so that they do not interfere with each other. Methods for efficiently identifying two antibodies that do not interfere with each other have not been established, making their preparation difficult. Furthermore, it becomes difficult to apply these methods to molecules with small molecular surface areas, such as small molecules. Non-Patent Document 4 describes a system that responds to intracellular miRNAs and cannot be applied to other molecules. Non-Patent Document 5 describes a system that is limited to extracellular molecules and cannot be applied to intracellular molecules.

It is necessary to establish a universal platform capable of specifically responding to various intracellular molecules in living cells.

### MEANS FOR SOLVING THE PROBLEM

As a result of intensive studies, the present inventors have conceived that, by expressing in cells a fusion protein in which a light-chain variable domain and a heavy-chain variable domain of an antibody that specifically recognizes a target molecule are respectively fused to the N-terminal and C-terminal fragments of a split functional protein, it would be possible to regulate the functional protein in response to the target molecule within a cell, and thus, have completed the present invention.

That is, the present invention includes the following.
[1] A target molecule-specific functional protein regulation system including: the following (a) and (b):
   (a) a first nucleic acid molecule including a nucleic acid sequence encoding a first fusion protein that includes a first target binding domain including a light-chain variable domain of an antibody that specifically recognizes a target molecule, and a first domain of a functional protein; and
   (b) a second nucleic acid molecule including a nucleic acid sequence encoding a second fusion protein that includes a second target binding domain including a heavy-chain variable domain of an antibody that specifically recognizes the target molecule, and a second domain of a functional protein; or

   the following (c) and (d):
      (c) the first fusion protein; and
      (d) the second fusion protein,
   wherein
      the first domain of the functional protein is either an N-terminal domain or a C-terminal domain of the functional protein, and the second domain of the functional protein is the other of the N-terminal domain or the C-terminal domain of the functional protein,
      a framework region of either the light-chain variable domain or the heavy-chain variable domain includes a sequence of a framework region of trastuzumab or a sequence of a framework region of a mutated sequence thereof, and
      the target molecule, the first target binding domain, and the second target binding domain form a ternary complex.
[2] The system according to [1], wherein the functional protein includes a detection protein, a protein that regulates transcription and translation of RNA, an extracellular secretory protein, a genome-editing protein, an enzyme, and a therapeutic protein.
[3] The system according to [1], further including (e) a third nucleic acid molecule regulated by the functional protein.
[4] The system according to [3], wherein the functional protein is a nucleic acid polymerase, and the third nucleic acid molecule includes a promoter sequence specific for the nucleic acid polymerase and a nucleic acid sequence encoding a protein of interest or an RNA of interest.
[5] The system according to [4], wherein the protein of interest includes a detection protein, a protein that regulates transcription or translation of RNA, an extracellular secretory protein, a genome-editing protein, an enzyme, and a therapeutic protein.
[6] The system according to [3], wherein the functional protein is a nucleic acid polymerase, and the third nucleic acid molecule includes a promoter sequence specific for the nucleic acid polymerase and a nucleic acid sequence encoding a guide RNA that specifically recognizes a genome-editing target molecule.
[7] The system according to [1], wherein the first or second nucleic acid molecule is a DNA construct or a synthetic mRNA molecule.
[8] A method for regulating a functional protein in a target molecule-specific manner within a cell, the method including a step of introducing the system according to [1] into a cell.
[9] The method according to [8], further including a step of introducing (e) a third nucleic acid molecule or additional protein regulated by the functional protein into the cell.

### EFFECTS OF THE INVENTION

According to the present invention, it becomes possible to detect any molecule, such as proteins, peptides, RNA, and small molecules, either inside cells or in cell-free extracts, enabling intracellular detection to be performed without disrupting the cells. It also enables cell regulation through gene expression, such as cell identification, separation/removal, and genome editing, following molecular detection. Furthermore, because these operations are completed solely by gene introduction into cells, the procedures are simplified compared to existing similar techniques. As further applications, the present invention enables the development of molecular detection methods and sensors for use in living cells, together with associated cell-sorting and cell-regulation techniques; methods for inducing gene expression specifically in target cells and gene-therapy technologies based thereon; and advances in the design of genetic circuit components in living cells and in cell manipulation and cell-engineering technologies utilizing such designs.

### BRIEF DESCRIPTION OF THE DRAWINGS

FIG. 1A is a schematic diagram illustrating the design and strategy of Target-dependent RNA polymerase (TdRNAP), where the split T7 RNAP assembles into a functional RNAP when the fused VH and VL domains interact with a target molecule. The activated RNAP can transcribe genes of interest (GOI) under the control of T7 promoter, resulting in various outputs and applications.
FIG. 1B schematically illustrates the plasmid used to test GCN4-dRNAP in 293FT cells. N-terminal and C-terminal split RNAP fragments (T7N and T7C) were fused to VL and VH domains of the anti-GCN4 antibody, respectively.
FIG. 1C shows fluorescence images of 293FT cells transfected with the GCN4-dRNAP and induced with EGFP or EGFP-GCN4. The scale bar represents 100 µm.
FIG. 1D shows dose-dependent transcriptional activation of GCN4-dRNAP. 293FT cells were transfected with the reporter plasmid encoding firefly luciferase and *Renilla* luciferase under the control of T7 and constitutive promoters, respectively. Subsequently, cells were then analyzed for luminescence. Values represent mean ± s.e. of n=3 biological replicates.
FIG. 1E shows the dependency of the antibody binding affinity on the transcription activity of GCN4-dRNAP (left panel), and CDR-H3 sequence and the reported dissociation constant (Kd) of each VH variant (right panel). Fold changes are calculated from the induction with EGFP-GCN4 versus EGFP for each VH variant. Values represent mean ± s.e. of n=3 biological replicates.
FIG. 2A shows the antibody substitution of TdRNAP using the anti-FLAG and anti-EGFP antibodies, resulting in FLAG- and EGFP-dRNAPs.
FIG. 2B illustrates the structural stabilization of the framework region of VL domain by CDR loop grafting and its impact on transcriptional activity. CDR loops for the VL domain of the anti-FLAG antibody were grafted to the mutated humanized trastuzumab framework region (CDR-grafted VL domain). Values represent mean ± s.e. of n=3 biological replicates, and statistical analysis was performed using an unpaired two-tailed t-test. *P < 0.05, **P < 0.01, and n.s. indicates no significant difference (P > 0.05).
FIG. 2C shows dose-and affinity-dependent transcriptional activation of FLAG-dRNAP. Values represent mean ± s.e. of n=3 biological replicates.
FIG. 2D shows target-specific and dose-dependent transcriptional activation of EGFP-dRNAP. Values represent mean ± s.e. of n=3 biological replicates.
FIG. 2E shows validation of the target specificity of GCN4-, FLAG-, and EGFP-dRNAPs by inducing with their corresponding target molecules. Values represent mean ± s.e. of n=3 biological replicates, and statistical analysis was performed using one-way ANOVA with Bonferroni correction. *P < 0.05, **P < 0.01, and n.s. indicates no significant difference (P > 0.05).
FIG. 3A shows the design of RNA and small-molecule-dependent RNAPs using the anti-HCV IRES RNA and anti-fluorescein antibodies.
FIG. 3B shows fluorescence images of 293FT cells transfected with HCV IRES RNA-dRNAP and induced with mono-cistronic or HCV IRES-containing bi-cistronic constructs. The scale bar represents 100 µm.
FIG. 3C shows a comparison of the tdTomato fluorescence intensity between cells induced with mono-cistronic and bi-cistronic constructs. The fluorescence intensity is quantified from the integrated density of tdTomato fluorescence. Values represent mean ± s.e. of n=3 biological replicates, and statistical analysis was performed using an unpaired two-tailed t-test. *P < 0.05, **P < 0.01, and n.s. indicates no significant difference (P > 0.05).
FIG. 3D shows fluorescence images of 293FT cells transfected with fluorescein-dRNAP and induced with 0, 50, or 100 µg/mL fluorescein. The scale bar represents 100 µm.
FIG. 3E shows dose-dependent transcriptional activation of Fluorescein-dRNAP. 293FT cells were transfected with the reporter plasmid encoding firefly luciferase and *Renilla* luciferase under the control of T7 and constitutive promoters, respectively. Values represent mean ± s.e. of n=3 biological replicates, and statistical analysis was performed using one-way ANOVA with Dunnett's multiple comparison test against the 0 µg/mL fluorescein, *P < 0.05.
FIG. 4A shows the design of a transcriptional amplification system using CGG RNAP as an additional output of GCN4-dRNAP. The system involves a T7 promoter-driven CGG RNAP plasmid and a dual promoter reporter plasmid encoding a luciferase reporter gene under the control of both T7 and CGG promoters. The activated GCN4-dRNAP induces the expression of both CGG RNAP and the reporter gene. The reporter gene expression is then further enhanced by the induced CGG RNAP.
FIG. 4B shows the validation of the enhancement of reporter expression by the amplification system in 293FT cells. Values represent mean ± s.e. of n=3 biological replicates.
FIG. 4C shows the design of an orthogonal genetic circuit consisting of EGFP-dependent T7 RNAP and GCN4-dependent CGG RNAP. Each TdRNAP independently regulates the expression of a luciferase reporter gene under its corresponding promoter. EGFP-dependent T7 RNAP and GCN4 dependent CGG RNAP correspond to the expression of firefly and *Renilla* luciferases, respectively.
FIG. 4D shows simultaneous monitoring of target-dependent reporter expressions in the same 293FT cells. RLU represents relative light units. Values represent mean ± s.e. of n=3 biological replicates.
FIG. 5A shows an application of TdRNAP to regulate genome editing in a target-dependent manner in human cells. GCN4-dRNAP induces guide RNA (gRNA) expression when cells express GCN4-fused EGFP (EGFP-GCN4). The gRNA targets and knocks out the EGFP gene by forming a complex with Cas9.
FIG. 5B shows an establishment of the 293FT cell lines carrying EGFP or EGFP-GCN4 gene in the AAVS1 locus. GCN4-dEGFP knockout should be triggered preferentially in the cells expressing EGFP-GCN4 than EGFP.
FIG. 5C shows flowcytometry analysis of the GCN4-dependent EGFP knockout in the established 293FT cell lines.
FIG. 5D shows an EGFP-negative cell population measured by flow cytometry in EGFP knockout experiments using T7 promoter for GCN4-dependent gRNA expression along with 200 ng of Cas9 expression plasmid. Values represent mean ± s.e. of n=7 biological replicates, and statistical analysis was performed using an unpaired two-tailed t-test. **P < 0.01, and n.s. indicates no significant difference (P > 0.05).
FIG. 5E shows an EGFP-negative cell population measured by flow cytometry in EGFP knockout experiments using U6 promoter for constitutive gRNA expression along with 200 ng of Cas9 expression plasmid. Values represent mean ± s.e. of n=5 biological replicates, and statistical analysis was performed using an unpaired two-tailed t-test. **P < 0.01, and n.s. indicates no significant difference (P > 0.05).

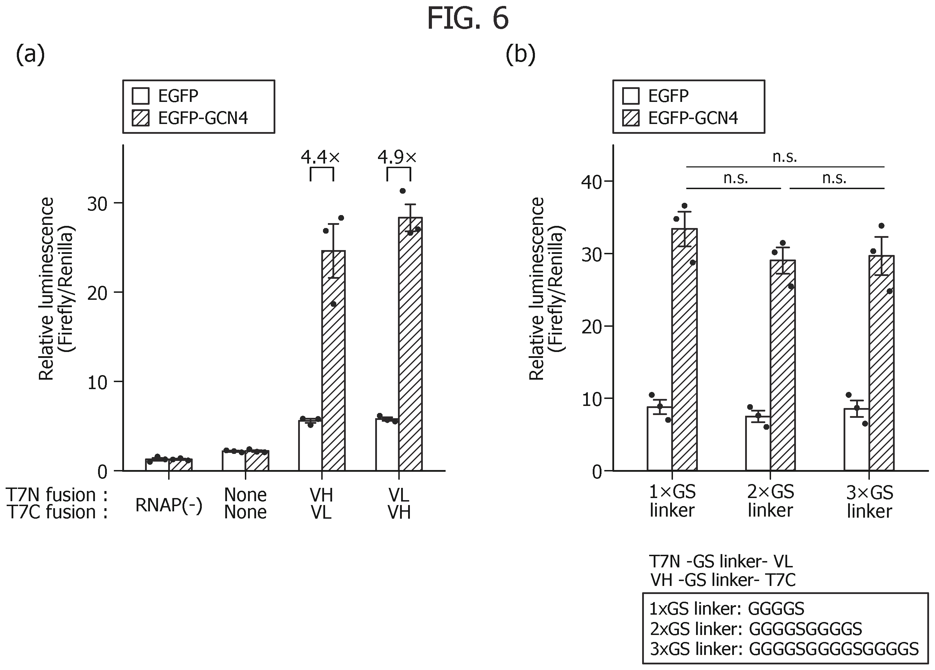

FIG. 6 shows an examination of the fusion patterns and linker lengths of TdRNAP. In FIG. 6(a), the fusion orientation of the VH domain and VL domain for each domain of the split T7 RNAP representing the transcription reaction of GCN4-dRNAP in 293FT cells is shown below the graph. Fifty induction plasmids were used, and each variable domain was fused to a 3×GS linker. RNAP(-) indicates transfection using an empty plasmid instead of split T7 RNAP fragments (T7N and T7C). FIG. 6(b) shows the transcriptional response of GCN4-dRNAP in 293FT cells, with the linker lengths between each variable domain and the two halves of split T7 RNAP varied as indicated in the figure. In both FIGs. 6(a) and 6(b), values represent mean ± s.e. of n=3 biological replicates, and statistical analysis was performed using one-way ANOVA with Bonferroni correction. The n.s. indicates no significant difference (P > 0.05).

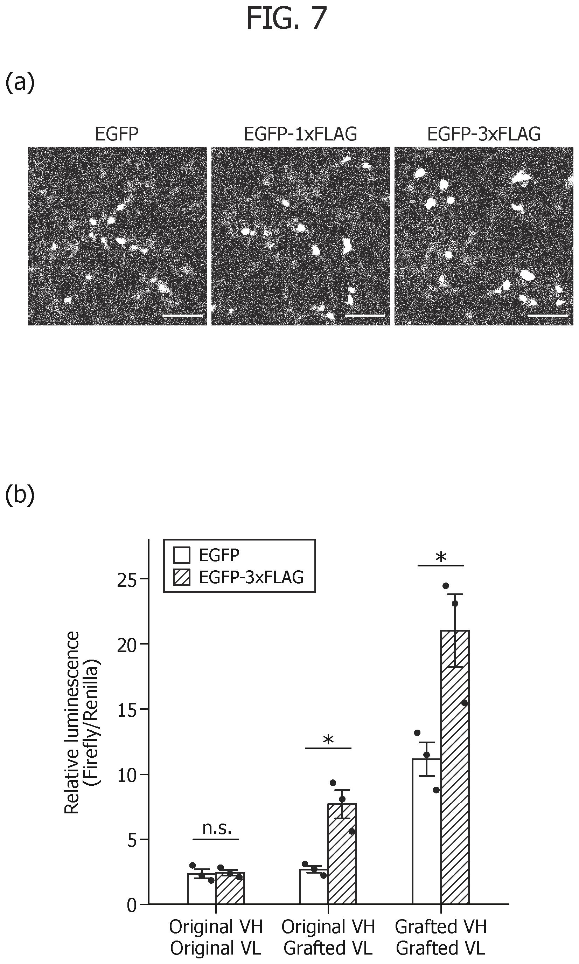

FIG. 7 shows the evaluation of FLAG-dRNAP with original and CDR loop graft variable domains. FIG. 7(a) shows fluorescence images of 293FT cells transfected with FLAG-dRNAP, which consists of the original VH and VL domains of the anti-FLAG antibody. The transfected cells were induced with EGFP, EGFP-1xFLAG, or EGFP-3xFLAG. The scale bar represents 100 µm. FIG. 7(b) shows the evaluation of the VH and VL domain frameworks by CDR loop grafting. CDR loops for the VH and VL domains of the anti-FLAG antibody were grafted to the framework of mutated humanized trastuzumab (grafted VH and VL). Values represent mean ± s.e. of n=3 biological replicates, and statistical analysis was performed using an unpaired two-tailed t-test. *P < 0.05, and n.s. indicates no significant difference (P > 0.05).

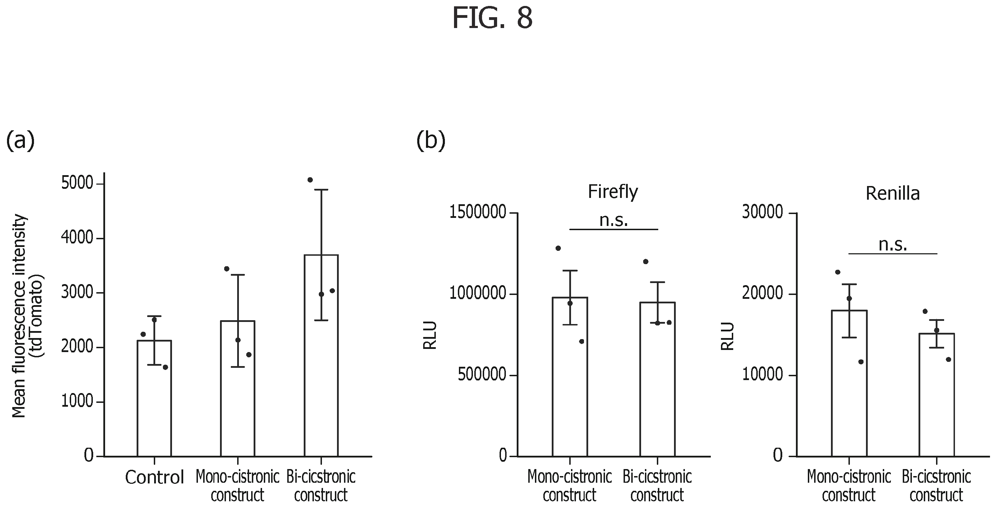

FIG. 8 shows the analysis of HCV IRES RNA-dependent transcriptional activation by HCV IRES RNA-dRNAP. FIG. 8(a) shows the mean tdTomato fluorescence intensity measured by flow cytometry. Transfected 293FT cells were induced with an empty plasmid (control), mono-cistronic construct, or a bi-cistronic construct. FIG. 8(b) shows the comparison of the luciferase expression levels between the mono-cistronic construct and the bi-cistronic construct. For FIGs. 8(a) and 8(b), values represent mean ± s.e. of n=3 biological replicates. For FIG. 8(b), statistical analysis was performed using an unpaired two-tailed t-test. The n.s. indicates no significant difference (P > 0.05).
FIG. 9A shows the design of Hsp70-dRNAP and its application for monitoring changes in endogenous Hsp70 expression levels. Transfected 293FT cells were stimulated at 42°C for 1 hour, leading to an increase in the expression level of endogenous Hsp70.
FIG. 9B shows the verification of Hsp70 promoter activation after heat shock stimulation using a plasmid encoding EGFP under the control of an Hsp70 promoter. The scale bar represents 100 µm.
FIG. 9C shows fluorescence images of stimulated and unstimulated 293FT cells transfected with Hsp70-dRNAP or split RNAP without VH and VL domains. The scale bar represents 100 µm.
FIG. 9D shows the comparison of the tdTomato fluorescence intensity in stimulated and unstimulated 293FT cells transfected with Hsp70-dRNAP or split RNAP without VH and VL domains. The fluorescence intensity is quantified from the integrated density of tdTomato fluorescence. Values represent mean ± s.e. of n=3 biological replicates, and the detection and exclusion of outliers were performed using the Grubbs' test (α = 0.05).

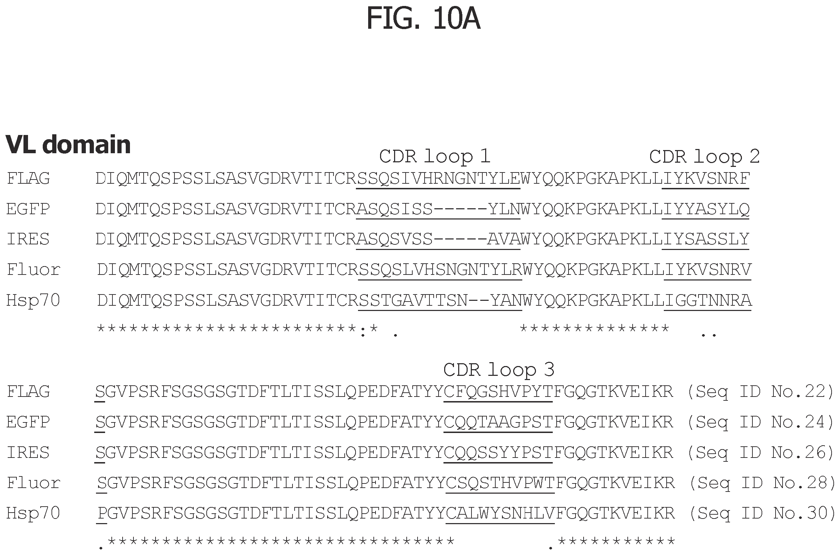

FIG. 10A shows the alignment of the first target binding domain (VL domain) used in the example.

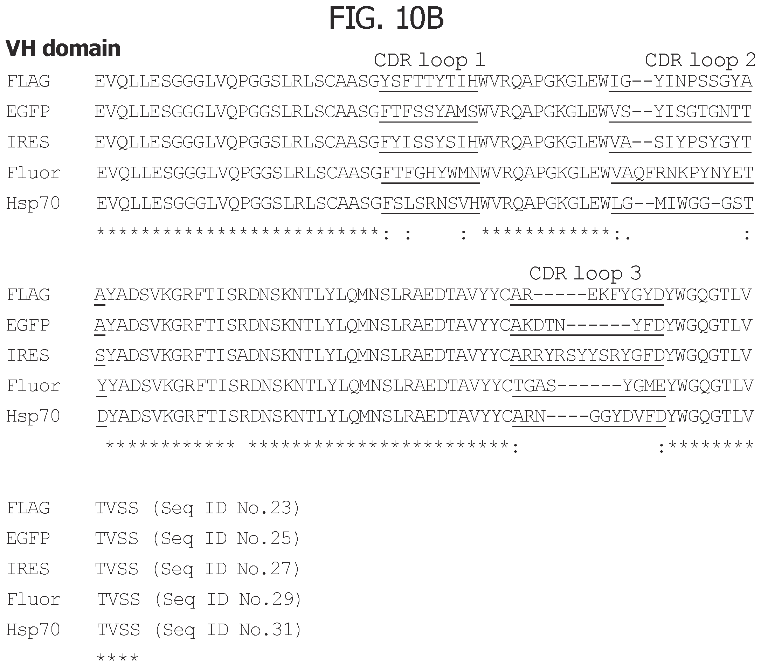

FIG. 10B shows the alignment of the second target binding domain (VH domain) used in the example.

### MODE FOR CARRYING OUT THE INVENTION

Hereinafter, embodiments of the present invention will be described. However, the present invention is not limited by the embodiment described below.

In one embodiment, the present invention relates to a target molecule-specific functional protein regulation system. The system includes
the following (a) and (b):
   (a) a first nucleic acid molecule including a nucleic acid sequence encoding a first fusion protein that includes a first target binding domain including a light-chain variable domain of an antibody that specifically recognizes a target molecule, and a first domain of a functional protein; and
   (b) a second nucleic acid molecule including a nucleic acid sequence encoding a second fusion protein that includes a second target binding domain including a heavy-chain variable domain of an antibody that specifically recognizes the target molecule, and a second domain of a functional protein; or
the following (c) and (d):
   (c) the first fusion protein; and
   (d) the second fusion protein,
wherein
   the first domain of the functional protein is either an N-terminal domain or a C-terminal domain of the functional protein, and the second domain of the functional protein is the other of the N-terminal domain or the C-terminal domain of the functional protein,
   a framework region of either the light-chain variable domain or the heavy-chain variable domain includes a sequence of a framework region of trastuzumab or a mutated sequence thereof, and
   the target molecule, the first target binding domain, and the second target binding domain form a ternary complex.

The system according to the present embodiment may optionally include the following (e):
(e) a third nucleic acid molecule regulated by the functional protein.

The term "target molecule-specific functional protein regulation system", in particular, to a system that regulates the activity of a functional protein in specific response to a target molecule. The phrase "that regulates the activity of a functional protein in specific response to a target molecule" means that the first and second domains of the functional protein are regulated so as to be reconstituted only when the target molecule is present, thereby allowing the functional protein to exhibit its inherent activity. More specifically, when the target molecule is absent, the functional protein remains inactive, and when the target molecule is present, the split functional protein regains its activity.

The term "target molecule" refers to a molecule that is present, at the time the system is used, within the system into which nucleic acid molecules (a) and (b) have been introduced, preferably within a cell; and, in certain embodiments, it may refer to a molecule present in the cytoplasm or nucleus of a target living cell. The target molecule is not particularly limited as long as it is a molecule that can be recognized by an antibody and that may be present transiently or constitutively within a cell. Specific examples of the target molecule include proteins, peptides, synthetic or natural low molecular compounds, synthetic or natural high molecular compounds, and compounds including nucleic acids such as DNA, RNA, and RNP, or fragments thereof. Depending on the purpose of the regulation, it is possible to selectively choose a target molecule as needed. For example, when aiming to regulate the expression of a functional protein according to intracellular conditions, any molecule that is specific to the intracellular condition to be detected, whether the molecule is a protein or a peptide, can be used. If the aim is to introduce a target molecule from outside the cell to achieve regulation, a low molecular compound that easily penetrates the cell membrane can be selected. Furthermore, it is possible to target metabolites that are generated as a result of natural or artificial reactions within cells, as target molecules. In this case, the metabolites may be low molecular compounds or the like. In another embodiment, the present system can also be operated in non-cellular environments, such as cell-free translation systems using microbial or cellular extracts, reaction systems in artificial cells using liposomes or the like, or in vitro molecular diagnostic assays, and the target molecule in such cases is not particularly limited.

In the present embodiment, the term "functional protein" refers to any protein that exerts a function in response to a target protein. The functional protein may include, but is not limited to a detection protein, a protein that regulates transcription or translation of RNA, an extracellular secretory protein, a genome-editing protein, an enzyme, and a therapeutic protein. These functional proteins may exert their functions independently, or may function in combination with other substances to exert detection, enzymatic activity, or therapeutic effects, or may act on other substances to elicit their functions. It should be noted that classifications such as detection proteins, genome-editing proteins, enzymes, and therapeutic proteins are for convenience of explanation, and a substance may fall under two or more of these classifications.

The term "detection protein" refers to any protein that has been translated and is capable of presenting detectable information. The detection protein may be a protein that can be visualized and quantified by fluorescence, luminescence, or color development, or by assisting such fluorescence, luminescence, or color development. Examples of the fluorescent protein include, but are not limited to, blue fluorescent proteins such as Sirius and EBFP; cyan fluorescent proteins such as mTurquoise, TagCFP, AmCyan, mTFP1, MidoriishiCyan, and CFP; green fluorescent proteins such as TurboGFP, AcGFP, TagGFP, Azami-Green (e.g., hmAG1), ZsGreen, EmGFP, EGFP, GFP2, and HyPer; yellow fluorescent proteins such as TagYFP, EYFP, Venus, YFP, PhiYFP, PhiYFP-m, TurboYFP, ZsYellow, and mBanana; orange fluorescent proteins such as Kusabira Orange (e.g., hmKO2) and mOrange; red fluorescent proteins such as TurboRFP, DsRed-Express, DsRed2, TagRFP, DsRed-Monomer, AsRed2, and mStrawberry; and far-red fluorescent proteins such as TurboFP602, mRFP1, JRed, KillerRed, mCherry, HcRed, KeimaRed (e.g., hdKeimaRed), mRasberry, and mPlum. Examples of the luminescent protein include, but are not limited to, Aequorin. Examples of the proteins that assist fluorescence, luminescence, or color development include, but are not limited to, enzymes such as luciferase, phosphatase, peroxidase, and β-lactamase, which degrade fluorescent, luminescent, or chromogenic precursors. When using an RNA molecule that contains, within the translation region, a nucleic acid sequence encoding a protein that assists fluorescence, luminescence, or color development, it is necessary to use it in a manner that allows a corresponding precursor and the protein translated from the RNA molecule to come into contact with each other. For example, a precursor substance may be brought into contact with cells into which the RNA molecule has been introduced, or the corresponding precursor substance may be introduced into the cells into which the RNA molecule has been introduced.

The term "genome-editing protein" refers to an enzyme that acts on a target gene to modify its functions. Modifying gene function includes, for example, reducing, eliminating, acquiring, or enhancing its function. More specifically, the term "editing" may refer to enzymes capable of cleaving genes, activating or suppressing gene expression, performing base editing, labeling (genome tagging), and conducting epigenome editing such as DNA methylation/demethylation and histone acetylation. Examples of such enzymes include nucleases and inactivated nucleases. Examples of the nucleases include, but are not limited to, Clustered Regularly Interspaced Short Palindromic Repeats-Associated Proteins 9 (Cas9), transcription activator-like effector nucleases (TALEN), homing endonucleases, and zinc finger nucleases. For both of the nucleases, it is also possible to use analogs possessing similar functions, namely cleavage activity toward a target gene, or derivatives having equivalent activity. For example, analogs of Cas9 protein include Cas9 family proteins, and specific examples thereof include, but are not limited to, *Streptococcus pyogenes* Cas9 (SpCas9), *Staphylococcus aureus* Cas9 (SaCas9), *Francisella novicida* Cas9 (FnCas9), *Campylobacter jejuni* (CjCas9), *Neisseria meningitidis* Cas9 (NmCas9 or NmeCas9), *Geobacillus stearothermophilus* Cas9 (GeoCas9), and *Streptococcus thermophilus* CRISPR1-Cas9 (St1Cas9). The nucleases also include fusion proteins in which other proteins are fused to Cas9 protein, or its analogs or its derivatives. Examples of the inactivated nuclease include an inactivated nuclease fused to a transcriptional activator. Specific examples include, but are not limited to, inactivated Cas9 (dCas9) nuclease fused to transcriptional activator VP64, such as dCas9-VP64, dCas9-VPR, dCas9-SunTag, dCas9-VP16, dCas9-VP160, and dCas9-P300.

Examples of the enzymes include, but are not limited to, nucleic acid polymerases, reverse transcriptases, regulatory proteins for transcription or translation, ribosomes, DNA/RNA modifying enzymes, oxidation-reduction enzymes, phosphorylating enzymes, and nucleic acid binding proteins. Genome-editing proteins are a type of enzyme, which can also be referred to as a genome editing enzyme. Examples of the nucleic acid polymerases include T7 RNA polymerase, Pfu DNA polymerase, and M-MLV reverse transcriptase. Examples of RNA transcribed by RNA polymerase include, but are not limited to, guide RNA, miRNA, siRNA, lincRNA, snoRNA, tRNA, and ribozyme. Examples of the oxidation-reduction enzymes include luciferase and dihydrofolate reductase enzymes. Examples of the phosphorylating enzymes include thymidine kinase. Examples of the nucleic acid binding proteins include endonucleases and PolyA-binding proteins.

Therapeutic protein refers to proteins that can be used for the treatment, prevention, and diagnosis of diseases and conditions by affecting the functions of cells. The phrase "affecting the function of a cell" includes increasing, decreasing, or maintaining a predetermined function of the cell within a certain range. Examples of the therapeutic protein can include, but are not limited to, a cell-proliferation protein, a cell-death protein, a cell signaling factor, a drug resistance gene, a transcriptional regulatory factor, a translational regulatory factor, a differentiational regulatory factor, a reprogramming induction factor, an RNA-binding protein factor, a chromatin regulator, a membrane protein, or fragments and complexes thereof. These proteins may also provide detectable information by influencing cellular functions, and thus can serve not only as therapeutic proteins but also as detection proteins. Examples of other therapeutic proteins include, but are not limited to, enzymes, growth factors, antibodies, antigens, proteins composing viruses or their components, proteins inhibiting virus production, genome-editing proteins, and fragments or complexes thereof. For example, a cell-proliferation protein functions as a marker by promoting the proliferation of only the cells that express it and thereby allow the proliferated cells to be identified. A cell-death protein functions as a marker by inducing cell death in the cells that express it, thereby killing cells that contain or do not contain a specific molecule (target substance), and indicating the viability of the cells. A cell-signaling factor functions as a marker by enabling the cells that express it to emit a specific biological signal, which can be identified to serve as the marker. Examples of the cell-death protein include, but are not limited to, RNA-degrading enzymes such as barnase derived from *Bacillus amyloliquefaciens*; toxins such as HokB, Fst, GhoT (membrane disruption), HipA (nucleic acid elongation inhibition via phosphorylation), RelE, YafO, VapC, MazF, MqsR, PemKHicA (endonuclease), FicT (Adenylation), OC (Phosphorylation), CcdB, ParE (Gyrase inhibitor), and Tact (Inhibitor of translation), cbtA (Inhibitor of cytoskeletal protein), and apoptosis-inducing proteins such as Bax and Bim. A translation-regulatory factor functions as a marker, for example, by recognizing and binding to the tertiary structure of a specific RNA and thereby regulating the translation of other mRNAs into proteins. Examples of the translation-regulatory factor include 5R1, 5R2 (Nat Struct Biol. 1998 Jul; 5(7): 543-6), B2 (Nat Struct Mol Biol. 2005 Nov; 12(11): 952-7), Fox-1(EMBO J. 2006 Jan 11; 25(1): 163-73.), GLD-1 (J Mol Biol. 2005 Feb 11; 346(1): 91-104.), Hfq (EMBO J. 2004 Jan 28; 23(2): 396-405), HuD (Nat Struct Biol. 2001 Feb; 8(2): 141-5.), SRP19 (RNA. 2005 Jul; 11(7): 1043-50), L1 (Nat Struct Biol. 2003 Feb; 10(2): 104-8.), L11 (Nat Struct Biol. 2000 Oct; 7(10): 834-7.), L18 (Biochem J. 2002 Mar 15; 362(Pt 3): 553-60), L20 (J Biol Chem. 2003 Sep 19; 278(38): 36522-30.), L23 (J Biomol NMR. 2003 Jun; 26(2): 131-7), L25 (EMBO J. 1999 Nov 15; 18(22): 6508-21.), L30 (Nat Struct Biol. 1999 Dec; 6(12): 1081-3.), LicT (EMBO J. 2002 Apr 15; 21(8): 1987-97.), MS2 coat (FEBS J. 2006 Apr; 273(7): 1463-75.), Nova-2(Cell. 2000 Feb 4; 100(3): 323-32), Nucleocapsid (J Mol Biol. 2000 Aug 11; 301(2): 491-511.), Nucleolin (EMBO J. 2000 Dec 15; 19(24): 6870-81.), p19 (Cell. 2003 Dec 26; 115(7): 799-811), L7Ae (RNA. 2005 Aug; 11(8): 1192-200.), PAZ (PiWi Argonaut and Zwille) (Nat Struct Biol. 2003 Dec; 10(12): 1026-32.), RnaseIII (Cell. 2006 Jan 27; 124(2): 355-66), RR1-38 (Nat Struct Biol. 1998 Jul; 5(7): 543-6.), S15 (EMBO J. 2003 Apr 15; 22(8): 1898-908.), S4 (J Biol Chem. 1979 Mar 25; 254(6): 1775-7.), S8 (J Mol Biol. 2001 Aug 10; 311(2): 311-24.), SacY (EMBO J. 1997 Aug 15; 16(16): 5019-29.), SmpB (J Biochem (Tokyo). 2005 Dec; 138(6): 729-39), snRNP U1A (Nat Struct Biol. 2000 Oct; 7(10): 834-7.), SRP54 (RNA. 2005 Jul; 11(7): 1043-50), Tat (Nucleic Acids Res. 1996 Oct 15; 24(20): 3974-81.), ThrRS (Nat Struct Biol. 2002 May; 9(5): 343-7.), TIS11d (Nat Struct Mol Biol. 2004 Mar; 11(3): 257-64.), Virp1 (Nucleic Acids Res. 2003 Oct 1; 31(19): 5534-43.), Vts1P (Nat Struct Mol Biol. 2006 Feb; 13(2): 177-8.), and λN (Cell. 1998 Apr 17; 93(2): 289-99.).

Therapeutic protein includes those that directly affect cellular function. Examples thereof can include, but are not limited to, a cell-proliferation protein, a cell-death protein, a cell signaling factor, a drug resistance gene, a transcriptional regulatory factor, a translational regulatory factor, a differentiational regulatory factor, a reprogramming induction factor, an RNA-binding protein factor, a chromatin regulator, and a membrane protein. For example, a cell-proliferation protein functions as a marker by promoting the proliferation of only the cells that express it, thereby allowing the proliferated cells to be identified. A cell-death protein functions as a marker by inducing cell death in the cells that express it, thereby killing cells, and indicating the viability of the cells. Examples of the cell death-protein include RNA-degrading enzyme Barnase, and apoptosis-promoting proteins such as Bax or Bim.

In a system according to the present embodiment, (a) a first nucleic acid molecule and (b) a second nucleic acid molecule are transcribed and translated in a cellular or cell-free translational system to express (c) a first fusion protein and (d) a second fusion protein. The system according to the present embodiment may include a combination of (a) a first nucleic acid molecule and (b) a second nucleic acid molecule, and/or a combination of (c) a first fusion protein and (d) a second fusion protein, or a combination of both. The first or second fusion protein can be designed in accordance with the target molecule and functional protein so as to suit the intended use of the present system. That is, the system can be designed such that, in the absence of a given target molecule, the functional protein remains in an inactive state, whereas upon INPUT of the target molecule, the functional protein regains its activity as an OUTPUT. For this purpose, the first fusion protein includes a first target binding domain including a light-chain variable domain (VL domain) of an antibody that specifically recognizes a target molecule, and a first domain of a functional protein. The second fusion protein includes a first target binding domain including a heavy-chain variable domain (VH domain) of an antibody that specifically recognizes the target molecule, and a second domain of a functional protein.

The first and second domains of the functional protein may be such that one is the N-terminal domain and the other is the C-terminal domain when the functional protein is divided into two parts. Thus, the first fusion protein may contain a C-terminal domain, and the second fusion protein may contain an N-terminal domain, or the first fusion protein may contain an N-terminal domain, and the second fusion protein may contain a C-terminal domain. There are no specific limitations on the sites in which a functional protein is divided into N-terminal domains and C-terminal domains, as long as the following conditions are met. That is, the conditions are that the N-terminal or C-terminal domain of the unreconstituted protein does not have the activity of the protein on its own, and that the functional protein is reconstituted by the VL domain and the VH domain of an antibody that specifically recognizes the target molecule. Here, the term "reconstitution" of a functional protein refers to a state in which the split functional protein is rendered capable of transitioning back to the original protein structure before division while maintaining the function (activity) of the functional protein.

The split site of the functional protein may, for example, be a loop region that does not form an α-helix or β-sheet. The site amenable to splitting of known functional proteins are generally well known from the literature and other sources, and one skilled in the art can determine the split site on the basis of such information. Alternatively, appropriate split sites can be confirmed through preliminary experiments or simulations conducted by those skilled in the art.

Next, the design of the first target binding domain and the second target binding domain will be explained. The first target binding domain includes the VL domain of an antibody that specifically recognizes the target molecule, while the second target binding domain includes the VH domain of an antibody that specifically recognizes the target molecule. The VL domain included in the first target binding domain and the VH domain included in the second target binding domain can be designed based on information obtained from monoclonal antibodies against the target molecule. Monoclonal antibodies against any target molecule can be obtained based on information in the literature, and some are commercially available. Furthermore, antibody gene sequences against the target molecule can be identified by antibody screening using phage display or by collecting the B-cell fraction from the peripheral blood of rabbits immunized with the target molecule. In addition, the antibodies can be designed and produced by in silico methods such as CDR-loop design using artificial intelligence or molecular docking simulations.

When the sequences of the VH domain and VL domain of a monoclonal antibody against the target molecule are obtained, these sequences can be directly included as the sequences constituting the first target binding domain and the second target binding domain in the first target binding domain and the second target binding domain. Alternatively, the sequence of either or both of the VH and VL domains of a monoclonal antibody against the target molecule may be modified and incorporated into the first and second target binding domains. When modified, at least one of the VH or VL domains is modified to include an amino acid sequence corresponding to the framework region of trastuzumab, preferably humanized trastuzumab, or a mutated sequence thereof. If the VH and VL domains of the monoclonal antibody against the target molecule inherently have an amino acid sequence corresponding to the framework region of trastuzumab, preferably humanized trastuzumab, or a mutated sequence thereof, no modification is required.

The VL domain and VH domain of monoclonal antibodies are each composed, in the direction from the N-terminus to the C-terminus, of the first framework region (FR1), the first complementarity-determining region (CDR loop 1), the second framework sequence (FR2), the second complementarity-determining region (CDR loop 2), the third framework region (FR3), the third complementarity-determining region (CDR loop 3), and the fourth framework region (FR4). In certain aspects of the present embodiment, in at least one of the VL domain and VH domain, at least one, preferably two, more preferably three, and most preferably all four of the four framework regions include an amino acid sequence corresponding to the respective framework regions of humanized trastuzumab, or a mutated sequence thereof. Furthermore, more preferably, all four framework regions consist of an amino acid sequence corresponding to the respective framework regions of humanized trastuzumab, or a mutated sequence thereof.

The amino acid sequence of the VL domain of humanized trastuzumab is shown in Seq ID No. 41, and the amino acid sequence of the VH domain is shown in Seq ID No. 42. In the amino acid sequence shown in Seq ID No. 41, FR1 is the amino acid sequence located at positions 1 to 24, FR2 is the amino acid sequence located at positions 35 to 47, FR3 is the amino acid sequence located at positions 57 to 87, and FR4 is the amino acid sequence located at positions 98 to 108. In the amino acid sequence shown in Seq ID No. 42, FR1 is the amino acid sequence located at positions 1 to 26, FR2 is the amino acid sequence located at positions 36 to 47, FR3 is the amino acid sequence located at positions 60 to 96, and FR4 is the amino acid sequence located at positions 109 to 120. It should be noted that the amino acid numbering described herein refers to numbering in which the N-terminus is designated as position 1, assuming that the full length of the VL domain is 108 amino acids and that of the VH domain is 120 amino acids.

For the designation of framework regions and CDR loops in antibodies, the Kabat numbering scheme may also be used. This is because the lengths of the CDR loops vary among antibodies, which can result in differences in the total amino acid length of each domain. FR1 to FR4 of the VL domain shown in Seq ID No. 41 according to the Kabat numbering are the same as those designated by the aforementioned numbering. According to the Kabat numbering, in the amino acid sequence shown in Seq ID No. 42, FR1 of the VH domain is the amino acid sequence located at positions 1 to 26, FR2 is the amino acid sequence located at positions 36 to 47, FR3 is the amino acid sequence located at positions 59 to 92, and FR4 is the amino acid sequence located at positions 102 to 113.

The amino acid sequence of a preferred variant of the VL domain of humanized trastuzumab (Seq ID No. 1) and the amino acid sequence of a preferred variant of the VH domain (Seq ID No. 2) are shown in Table 1 below. Hereinafter, the present invention will be described by way of example using the preferred mutated sequences shown in Seq ID Nos. 1 and 2. These sequences are referred to herein as "mutated humanized trastuzumab sequences". In both the VL domain and the VH domain, the underlined portions represent the complementarity determining regions, which sequentially denote CDR loop 1, CDR loop 2, and CDR loop 3 starting from the N-terminal side. FR1 represents the region on the N-terminal side of CDR loop 1; FR2 represents the region on the C-terminal side of CDR loop 1 and the N-terminal side of CDR loop 2; FR3 represents the region on the C-terminal side of CDR loop 2 and the N-terminal side of CDR loop 3; and FR4 represents the region on the C-terminal side of CDR loop 3. In the framework regions, residues shown in bold indicate amino acid residues that are preferably not substituted, and residues enclosed by boxes indicate amino acid residues that may be substituted.

**[Table 1]**

| |
|---|
| mutated humanized Trastuzumab VL(Seq ID No. 1) |
| DIQMTQSPSS LSASVGDRVT ITCRASQDVN TAVAWYQQKP GKAPKLLIYS ASFL YSGVPS RFSGSGSGTD FTLTISSLQP EDFATYYCQQ HYTTPPTFGQ GTKVEIKR |
| mutated humanized Trastuzumab VH (Seq ID No. 2) |
| EVQLLESGGG LVQPGGSLRL SCAASGFNIK DTYIHWVRQA PGKGLEWVAR IYPT NGYTRY ADSVKGRFTI SRDNSKNTLY LQMNSLRAED TAVYYCSRWG GDGFYAM DYW GQGTLVTVSS |

Accordingly, the first target binding domain of the present embodiment includes a VL domain of an antibody that specifically recognizes a target molecule, provided that FR1 of the VL domain preferably includes a sequence of FR1 of a VL domain of a mutated humanized trastuzumab (DIQMTQSPSS LSASVGDRVT ITCR (Seq ID No. 3)), and/or FR2 of the VL domain preferably includes a sequence of FR2 of a VL domain of a mutated humanized trastuzumab (WYQQKP GKAPKLL (Seq ID No. 4)), and/or FR3 of the VL domain preferably includes a sequence of FR3 of a VL domain of a mutated humanized trastuzumab (GVPS RFSGSGSGTD FTLTISSLQP EDFATYY (Seq ID No. 5)) and/or FR4 of the VL domain preferably includes a sequence of FR4 of a VL domain of a mutated humanized trastuzumab (FGQ GTKVEIKR (Seq ID No. 6)).

The second target binding domain of the present embodiment includes a VH domain of an antibody that specifically recognizes the target molecule. However, it is preferable that FR1 of the VH domain include the FR1 sequence of the mutated humanized trastuzumab VH domain (EVQLLESGGG LVQPGGSLRL SCAASG; Seq ID No. 7), and/or that FR2 of the VH domain include the FR2 sequence of the mutated humanized trastuzumab VH domain (WVRQA PGKGLEW; Seq ID No. 8), and/or that FR3 of the VH domain include the FR3 sequence of the mutated humanized trastuzumab VH domain (Y ADSVKGRFTI SRDNSKNTLY LQMNSLRAED TAVYYC; Seq ID No. 9), and/or that FR4 of the VH domain includes the FR4 sequence of the mutated humanized trastuzumab VH domain (DYW GQGTLVTVSS; Seq ID No. 10). However, another embodiment is also possible in which the FR3 sequence of the mutated humanized trastuzumab VH domain is the sequence Y ADSVKGRFTI SADNSKNTLY LQMNSLRAED TAVYYC (Seq ID No. 11).

The sequence of the framework regions of the VL domain and VH domain of the mutated humanized trastuzumab (Seq ID Nos. 3 to 11) may be partially substituted. For example, up to about 50%, preferably up to about 20%, and more preferably up to about 10% of each sequence in the framework regions may be substituted.

In certain embodiments, the amino acid sequences of the VL domain of the mutated humanized trastuzumab may each independently have approximately 5% substitution in FR1, approximately 47% substitution in FR2, approximately 17% substitution in FR3, and approximately 10% substitution in FR4. The amino acid sequences of the VH domain may each independently have approximately 16% substitution in FR1, approximately 9% substitution in FR2, approximately 46% substitution in FR3, and approximately 9% substitution in FR4.

In another embodiment, the amino acid residues indicated in enclosed text in each framework region of the sequences shown in Seq ID Nos. 1 and 2 may be substituted, and the substituted amino acid residues may be any amino acid residue. The amino acid residues indicated in bold should preferably not be substituted.

For both the VL domain of the first target binding domain and the VH domain of the second target binding domain, the CDR loops can be determined by numbering the amino acid sequences of the VH and VL domains of the antibody against the target molecule according to the Kabat numbering. Specifically, CDR loop 1 of the VL domain of the first target binding domain may be an amino acid sequence located at positions 25-34 of the VL domain of the antibody against the target molecule, and/or CDR loop 2 may be an amino acid sequence located at positions 48-56 of the VL domain of the antibody against the target molecule, and/or CDR loop 3 may be an amino acid sequence located at positions 88-97 of the VL domain of the antibody against the target molecule. Accordingly, at least one, preferably two, and more preferably all three of CDR loop 1, CDR loop 2, and CDR loop 3 of the VL domain of the first target binding domain may include a sequence derived from the VL domain of the antibody against the target molecule as designated by the Kabat numbering. In addition, up to about 20%, preferably up to about 10%, and more preferably up to about 5% of these sequences may be substituted.

Similarly, CDR loop 1 of the VH domain of the second target binding domain may be an amino acid sequence located at positions 27-35 of the VH domain of the antibody against the target molecule, and/or CDR loop 2 may be an amino acid sequence located at positions 48-58 of the VH domain of the antibody against the target molecule, and/or CDR loop 3 may be an amino acid sequence located at positions 93-101 of the VH domain of the antibody against the target molecule. Accordingly, at least one, preferably two, and more preferably all three of CDR loop 1, CDR loop 2, and CDR loop 3 of the VH domain of the second target binding domain may include a sequence derived from the VH domain of the antibody against the target molecule as designated by the Kabat numbering. In addition, up to about 20%, preferably up to about 10%, and more preferably up to about 5% of these sequences may be substituted.

In both the VL and VH domains, the lengths of the CDR loops are not particularly limited; for example, they may be about 3 to 30 amino acids in length, may be about 5 to 20 amino acids in length, or may be about 8 to 15 amino acids in length.

The first target binding domain includes a VL domain, but preferably does not include a constant domain or VH domain. Furthermore, the second target binding domain includes a VH domain, but preferably does not include a constant domain or VL domain. Furthermore, the first and the second target binding domains do not include the intermolecular disulfide bond that is normally present in the VH and VL domains of monoclonal antibodies.

In addition to having the structural features, the first target binding domain and the second target binding domain form a ternary complex with the target molecule. The phrase "form a ternary complex" is defined as a state in which, in the presence of a target molecule, the first target binding domain and the second target-binding domain each bind to the same target molecule, thereby stably maintaining the interaction between the first and second target binding domains. Preferably, the ternary complex has an equilibrium dissociation constant Kd of 50 nM or less. The equilibrium dissociation constant Kd is more preferably 10 nM or less, and most preferably 0.6 nM or less. The equilibrium dissociation constant Kd of the ternary complex can be determined by surface plasmon resonance (SPR) or isothermal titration calorimetry (ITC).

It is possible to design the first fusion protein and the second fusion protein by combining the domains designed as described above. The combination of the first fusion protein and the second fusion protein that function in the system according to the present embodiment can be as shown in Table 2 below. In Table 2 below, each domain is listed from left to right in the order from the N-terminus to the C-terminus of the fusion protein. However, when the functional protein is T7 RNA polymerase, it is preferable to use combination number 1 or 3 shown in Table 2 below.

**[Table 2]**

| Combination No. | First fusion protein | Second fusion protein |
|---|---|---|
| 1 | [First target binding domain]-[N-terminal domain] | [Second target binding domain]-[C-terminal domain] |
| 2 | [N-terminal domain-[First target binding domain] | [C-terminal domain]-[Second target binding domain] |
| 3 | [First target binding domain]-[C-terminal domain]- | [Second target binding domain]-[N-terminal domain] |
| 4 | [C-terminal domain]-[First target binding domain] | [N-terminal domain-[Second target binding domain] |

In this manner, the first domain (F1) of the functional protein and the first target binding domain (VL), which includes the VL domain of an antibody, can be selected or designed, and the first fusion protein can be designed and its structure determined. Similarly, the second target binding domain (VH), which includes the VH domain of an antibody, and the second domain (F2) of the functional protein can be selected or designed, and the second fusion protein can be designed and its structure determined. In the first and second fusion proteins, the domains may be directly linked to each other or may be linked via a linker. The lengths of the linker are not particularly limited, but, for example, may be about 3 to 50 amino acids in length, may be about 3 to 30 amino acids in length, or may be about 3 to 15 amino acids in length. In addition, the amino acid sequence of the linker is not particularly limited as long as the linker itself does not undergo self-association, does not possess strong polarity that could interfere with the formation of the ternary complex among VH, VL, and the target molecule, and is not cleaved either spontaneously or enzymatically. Furthermore, in the first and second fusion proteins, additional amino acid sequences of about 3 to 200 amino acids in length may be included at the N-terminal or C-terminal side of each domain.

Once the structures of (c) the first fusion protein and (d) the second fusion protein have been determined, the structures of (a) the first nucleic acid molecule and (b) the second nucleic acid molecule, which express these fusion proteins, can be determined. The first nucleic acid molecule (a) and the second nucleic acid molecule (b) may each independently be a DNA construct or a synthetic RNA molecule, and either may be appropriately used depending on the purpose and application of the present invention.

The DNA construct may, for example, employ vectors that are well known and commonly used in the art, such as viral vectors, artificial chromosome vectors, plasmid vectors, or expression systems using transposons (sometimes referred to as transposon vectors). Examples of the virus vectors include retrovirus vectors, lentivirus vectors, adenovirus vectors, adeno-associated virus vectors, and Sendai virus vectors. Examples of the artificial chromosome vectors include human artificial chromosome (HAC), yeast artificial chromosome (YAC), and bacterial artificial chromosome (BAC, PAC). As plasmid vectors, any plasmids for use in mammalian cells may be employed, and, for example, episomal vectors may be used. Examples of transposon vectors include expression vectors utilizing the piggyBac transposon. A DNA construct may also be designed such that the first and second fusion proteins are expressed as separate proteins from a single expression vector.

The synthetic RNA molecules may be a combination of two mRNA molecules: one mRNA molecule including, in the 5' to 3' direction, a 5'-UTR, an open reading frame containing a nucleic acid sequence encoding the first fusion protein, and a 3'-UTR; and another mRNA molecule including, in the 5' to 3' direction, a 5'-UTR, an open reading frame containing a nucleic acid sequence encoding the second fusion protein, and a 3'-UTR. Alternatively, the synthetic RNA molecule may be a single mRNA molecule including, in the 5' to 3' direction, a 5'-UTR; an open reading frame containing a nucleic acid sequence encoding the second fusion protein, a self-cleaving sequence encoding a self-cleaving peptide, and a nucleic acid sequence encoding the first fusion protein; and a 3'-UTR.

The 5' UTR of the mRNA contains a cap structure or a cap analog at the 5' end. The cap structure may be 7-methylguanosine-5'-phosphate, and the cap analog is a modified structure that is recognized by the translation initiation factor eIF4E in the same manner as the cap structure. Examples include, but are not limited to, Anti-Reverse Cap Analog (ARCA) manufactured by Ambion, the m7G(5')ppp(5')G RNA Cap Structure Analog manufactured by New England Biolabs, and CleanCap manufactured by TriLink. The cap analog may also be any other modified structure that is recognized by a translation initiation factor. On the 3' side of the cap structure or cap analog and on the 5' side of the start codon, an optional nucleic acid sequence may be included, for example, of about 0 to 500 bases, preferably about 20 to 200 bases. It is preferable that such optional nucleic acid sequences do not form secondary structures and do not specifically interact with other nucleic acid molecules. It is also preferable that no AUG serving as a start codon be present within the 5' UTR.

The open reading frame, which is the coding region of the mRNA, includes a start codon, a nucleic acid sequence encoding the first or second fusion protein, and a stop codon. The nucleic acid sequence encoding the first fusion protein includes, from the 3' side of the start codon AUG toward the 5' side of the stop codon, a nucleic acid sequence encoding the N-terminal domain of the fusion protein, a nucleic acid sequence encoding an optional linker amino acid sequence, and a nucleic acid sequence encoding the C-terminal domain of the fusion protein, in this order. The nucleic acid sequences encoding each domain can be determined from the previously designed structure of the first fusion protein.

The 3' UTR of the mRNA contains a PolyA tail. The PolyA tail may be a sequence containing about 50 to 250 adenine bases. However, it is not necessary that about 50 to 250 adenine bases be continuously linked, and other nucleic acid bases may be included between the adenine bases as long as the stability of the mRNA can be maintained.

The mRNAs described above may each have sugar residues (ribose) of the nucleotides modified, for example, for the purpose of reducing cytotoxicity. Examples of sites on the sugar residue that may be modified include the hydroxyl group or hydrogen atom at the 2', 3', and/or 4' positions, which may be replaced with other atoms. Examples of types of modifications include, for example, fluorination; alkoxylation (e.g., methoxylation, ethoxylation); O-allylation; S-alkylation (e.g., S-methylation, S-ethylation); S-allylation; and amination (e.g., -NH₂). Such modifications of the sugar residues can also be carried out by methods known in the art (see, for example, Sproat et al., (1991) Nucle. Acid. Res. 19, 733-738; Cotton et al., (1991) Nucl. Acid. Res. 19, 2629-2635; Hobbs et al., (1973) Biochemistry 12, 5138-5145).

The sugar residues of the mRNA may also be a bridged nucleic acid (BNA; also referred to as linked nucleic acid, LNA), in which a cross-linked structure is formed between the 2' and 4' positions. Such modifications of the sugar residues can also be carried out by methods known in the art (see, for example, Tetrahedron Lett., 38, 8735-8738 (1997); Tetrahedron, 59, 5123-5128 (2003); and Rahman S.M.A., Seki S., Obika S., Yoshikawa H., Miyashita K., Imanishi T., J. Am. Chem. Soc., 130, 4886-4896 (2008)). The mRNA may also contain nucleic acid bases (e.g., purines or pyrimidines) that are modified, for example, by chemical substitution. Examples of such modifications include 5-position pyrimidine modifications; 6- and/or 8-position purine modifications; modifications at exocyclic amines; substitution with 4-thiouridine; and substitution with 5-bromo- or 5-iodo-uracil. For the purpose of reducing cytotoxicity, the mRNA may contain modified bases such as pseudouridine (Ψ), N1-methylpseudouridine (N1mΨ), or 5-methylcytidine (5mC) in place of normal uridine or cytidine. The positions of the modified bases may, in either uridine or cytidine, independently be at all positions or be only at some positions thereof, and when only some are modified, the modifications may be introduced at random positions in any desired proportion.

To enhance resistance to nucleases and hydrolysis, for example, the phosphate groups contained in the mRNA (e.g., terminal phosphate residues) may be modified. For example, the phosphate group, i.e., the P(O)O group, may be substituted with P(O)S (thioate), P(S)S (dithioate), P(O)NR₂ (amidate), P(O)R, R(O)OR', CO, or CH₂ (formyl acetal), or with a 3'-amine (-NH-CH₂-CH₂-), where, each R or R' is independently hydrogen or a substituted or unsubstituted alkyl group (e.g., methyl or ethyl). Examples of linking groups include -O-, - N-, and -S-, through which the nucleotide can be bonded to an adjacent nucleotide.

Once the molecular structure and nucleic acid sequence of the DNA construct or mRNA have been determined as described above, they can be synthesized by one skilled in the art using any gene-engineering method known in the art. For example, mRNA can be obtained as a synthetic mRNA molecule by an in vitro transcription method using a template DNA containing a promoter sequence as the template. Furthermore, it is possible to obtain a first fusion protein (c) and a second fusion protein (d) from these DNA constructs or mRNA.

The third nucleic acid molecule (e), which may be optionally included in the system according to the present embodiment, is a nucleic acid that is regulated by the functional protein and produces a coding or non-coding RNA, or a desired protein. The third nucleic acid molecule can be designed in relation to the functional proteins encoded by the first and second nucleic acid molecules. Furthermore, additional proteins that may be optionally included in the system according to the present embodiment may be proteins regulated by functional proteins.

For example, when the functional protein is a nucleic acid polymerase, it is preferred that the third nucleic acid molecule include a promoter sequence specific for the nucleic acid polymerase and a nucleic acid sequence encoding a protein of interest or an RNA of interest. The protein of interest and RNA of interest are not particularly limited and may be selected and designed so as to be compatible with the purpose of the system of the present embodiment. The protein of interest may be selected, for example, from the functional proteins exemplified above, and from proteins that regulate gene expression, including transcription factors and translation factors; proteins applicable to vaccines, such as viral proteins or pathogenic proteins; or proteins applicable to cell selection, such as drug-degrading proteins or cell-death-inducing proteins. The RNA of interest may be, for example, a guide RNA (gRNA) that specifically recognizes a molecule to be subjected to genome editing; a switch mRNA that regulates translation in response to a specific miRNA or protein; or an shRNA, tRNA, or rRNA. The promoter sequence specific for the nucleic acid polymerase is also not particularly limited, and any known combination may be used. For example, combinations such as T7 polymerase with a T7 promoter, or a mutated T7 polymerase with its corresponding promoter, may be used, but are not limited thereto. In this case, the third nucleic acid molecule can produce the protein of interest or RNA of interest in response specifically to the reconstitution of the nucleic acid polymerase.

When the functional protein is a nucleic acid polymerase and the third nucleic acid molecule is a gRNA that specifically recognizes the molecule to be genome-edited, the system may include, as the fourth nucleic acid molecule, a nucleic acid containing a sequence encoding a genome-editing protein. In this system, in response to the target molecule, the first fusion protein and the second fusion protein assemble, thereby reconstituting the nucleic acid polymerase. The nucleic acid polymerase then produces gRNA, and the gRNA cooperates with the genome-editing protein produced by the fourth nucleic acid molecule to carry out genome editing of the target molecule.

For instance, when the functional protein is a genome-editing protein, it is preferable that the third nucleic acid molecule contain a nucleic acid sequence encoding a gRNA that specifically recognizes the molecule targeted for genome editing. In this case, the third nucleic acid molecule can, in response specifically to the reconstitution of the genome-editing protein, cleave the target molecule, or alternatively, inactivate another genome-editing protein that is already functioning.

For example, when the functional protein is an RNA-binding protein, it is preferable that the third nucleic acid molecule contain a nucleic acid sequence encoding a switch mRNA containing a nucleic acid sequence that specifically recognizes the RNA-binding protein. In this case, the third nucleic acid molecule may be a molecule that, in response specifically to the reconstitution of the RNA-binding protein, binds to the switch mRNA and is capable of promoting or suppressing translation from the switch mRNA.

The third nucleic acid molecule may be either a DNA construct or an mRNA, although depending on the purpose it may be necessary to be a DNA construct. Once the molecular structure and nucleic acid sequence of the DNA construct or mRNA have been determined, the third nucleic acid molecule can also be synthesized by one skilled in the art, in the same manner as the first and second nucleic acid molecules described above, using any gene-engineering method known in the art.

Next, the present invention is described from the viewpoint of the method. The method according to the present embodiment is a method for regulating a functional protein that is specific to a target protein, the method including a step of introducing the first and second nucleic acid molecules included in the aforementioned system into a cellular or cell-free translation system. Optionally, the method may further include a step of introducing a third nucleic acid molecule regulated by the functional protein into a cellular or cell-free translation system.

In the method according to the present embodiment, the term "cell" is not particularly limited and may be any cell. The cell may be a single cell, or may be a "cell population", which is an aggregate of two or more cells. There is no theoretical upper limit to the number of cells in a "cell population", and the term refers to a population composed of any number of cells. For example, the "cell population" may be a population containing multiple types of different cells, and may include both target cells and non-target cells.

The "cell" may be a cell derived from a unicellular or multicellular organism, and may also be a cell that has been subjected to artificial manipulation (including a cell line). For example, yeast, insect cells, or animal cells may be used, with animal cells being preferred. Examples of animal cells include, for example, cells derived from mammals such as mice, rats, hamsters, guinea pigs, dogs, monkeys, orangutans, chimpanzees, and humans. Examples of cells derived from mammals include cell lines such as monkey COS-7 cells, monkey Vero cells, Chinese hamster ovary (CHO) cells, dhfr-deficient CHO cells, mouse L cells, mouse AtT-20 cells, mouse myeloma cells, rat GH3 cells, human fetal kidney-derived cells (e.g., HEK293 cells), human hepatocellular carcinoma-derived cells (e.g., HepG2), and human FL cells. Primary cultured cells prepared from tissues of humans or other mammals may also be used. Furthermore, zebrafish embryos and Xenopus oocytes may also be used.

There are no particular limitations on the degree of differentiation of the cells or on the age of the animal from which the cells are obtained, and the cells may be any of (A) stem cells, (B) precursor cells, (C) fully differentiated somatic cells, or (D) other types of cells. Examples of stem cells (A) include, but are not limited to, embryonic stem (ES) cells, embryonic stem (ntES) cells derived from cloned embryos obtained by nuclear transfer, spermatogonial stem cells (GS cells), embryonic germ cells (EG cells), and induced pluripotent stem (iPS) cells. Examples of precursor cells (B) include tissue-specific stem cells (somatic stem cells) such as neural stem cells, hematopoietic stem cells, mesenchymal stem cells, and dental pulp stem cells. Examples of somatic cells (C) include keratinizing epithelial cells (e.g., keratinizing epidermal cells); mucosal epithelial cells (e.g., epithelial cells of the tongue surface); exocrine gland epithelial cells (e.g., mammary gland cells); hormone-secreting cells (e.g., adrenal medullary cells); metabolic or storage cells (e.g., hepatocytes); luminal epithelial cells forming boundary surfaces (e.g., type I alveolar cells); luminal epithelial cells of internal ducts (e.g., vascular endothelial cells); ciliated cells with transport capability (e.g., airway epithelial cells); extracellular matrix-secreting cells (e.g., fibroblasts); contractile cells (e.g., smooth muscle cells); blood and immune system cells (e.g., T lymphocytes); sensory cells (e.g., rod cells); neurons and glial cells of the central and peripheral nervous systems (e.g., astrocytes); pigment cells (e.g., retinal pigment epithelial cells); and their precursor cells (tissue progenitor cells). Examples of other cells (D) include cells that have undergone induced differentiation, and also include precursor cells and somatic cells differentiated from pluripotent stem cells. The cells may also be those induced by so-called "direct conversion" (also referred to as direct reprogramming or trans-differentiation), in which somatic cells or precursor cells are directly differentiated into desired cells without passing through an undifferentiated state.

The introduction into the cell of (a) the first nucleic acid molecule and (b) the second nucleic acid molecule, and/or (c) the first fusion protein and (d) the second fusion protein, and the introduction into the cell of the optionally usable third nucleic acid molecule or additional protein molecules, are described below. In the method for regulating a functional protein according to the present embodiment, the introduction of the first and second nucleic acid molecules into the cell may be carried out in vitro or in vivo, and is not particularly limited. The first and second nucleic acid molecules may be introduced separately into the cell, or they may be introduced as a single vector or RNA molecule. When using the third nucleic acid molecule, it is possible to introduce the first, second, and third nucleic acid molecules into cells separately or introduce them into cells as a single vector or RNA molecule.

For in vitro introduction, any method commonly used for introducing DNA constructs, RNA, or fusion proteins or additional proteins into cells in vitro may be employed. Examples of such introduction methods include, but are not limited to, lipofection, polymer-based methods, electroporation, calcium phosphate co-precipitation, DEAE-dextran methods, microinjection, and gene gun methods. Introduction of the first and second fusion proteins into the cell may be carried out, for example, by performing transfection using protein-delivery reagents such as cationic lipids, cell-penetrating peptides, polymer capsules, or polymer nanogels. Introduction into cells in vivo may be performed using any method commonly employed for introducing RNA into cells in vivo. For example, in mammals, DNA constructs, RNA, or the first and second fusion proteins can be directly introduced into cells by using introduction methods such as intramuscular injection, subcutaneous injection, intravenous injection, or intra-articular injection.

The amounts of the first and second nucleic acid molecules, the first and second fusion proteins, and the optionally used third nucleic acid molecule or additional proteins to be introduced into the cell are not limited to any particular values, as they vary depending on factors such as the type of target cell and the structure of the DNA or RNA. An amount of introduction sufficient to achieve the desired regulation can be determined through preliminary experiments or the like. Furthermore, the introduction of the first and second nucleic acid molecules, the first and second fusion proteins, and the optional third nucleic acid molecule or additional protein into a cell-free translation system can be achieved by adding the first and second nucleic acid molecules, the first and second fusion proteins, and the optional third nucleic acid molecule or additional protein to a cell-free translation system capable of performing any cell-free translation using any method.

The first and second nucleic acid molecules introduced into cells produce the first fusion protein and the second fusion protein within the cells. Furthermore, the first and second fusion proteins introduced into the cells remain present within the cells as the first and second fusion proteins, respectively. When the target molecule is present within the cell, the first target binding domain, the second target binding domain, and the target molecule form a specific ternary complex. As a result, the N-terminal domain and the C-terminal domain of the functional protein are reconstituted. The reconstituted functional protein exerts its intended function in the cells. For example, the reconstituted functional protein can acquire polymerase activity to initiate RNA synthesis, emit fluorescence, or possess genome-editing activity to initiate genome editing. On the other hand, when a target molecule is not present within the cells, the first target binding domain and the second target binding domain do not interact with each other, and the first fusion protein and the second fusion protein exist within the cells as separate proteins. That is, the functional protein is not reconstituted. When the first fusion protein and the second fusion protein are not reconstituted, the functional protein does not act together with, or on, the optional third nucleic acid molecule or additional protein.

### EXAMPLES

Hereinafter, the present invention will be described in more detail with the examples of the present invention. However, the examples below do not limit the present invention.

### [Experimental Methods]

### Plasmid construction

The plasmids for each TdRNAP were constructed by HiFi assembly (NEB) with PCR products or a Kunkel mutagenesis method with a pre-constructed plasmid. The genes for N-terminal and C-terminal fragments of T7 RNAP were amplified from plasmid pCAG-T7pol (Addgene plasmid #59926). The gene for the evolved T7 RNAP N-terminal fragment, d5-19, was generated by multiple site-directed mutagenesis. The genes for VH and VL domains of anti-GCN4 antibody were amplified from plasmid pHR-scFv-GCN4-sfGFP-GB1-NLS-dWPRE (Addgene plasmid #60906). The genes for anti-EGFP and anti-FLAG antibodies were amplified from synthetic DNA fragments (Thermo Fisher Scientific). The genes for anti-HCV IRES RNA, anti-fluorescein, and anti-Hsp70 antibodies were generated by the Kunkel mutagenesis method using the anti-FLAG antibody gene as the template DNA. All plasmids were constructed by HiFi assembly with PCR products and are listed in Tables 3A, 3B, and 3C. All the standard and mutagenic PCR were conducted using PrimeSTAR Max DNA Polymerase (Takara Bio). Seq ID Nos. of sequences of the individual proteins and peptides are as listed in Table 4, and the detailed sequences are provided in the Sequence Listing. Seq ID Nos. of nucleotide sequences of the key genes and regulatory elements used in the present study are also as listed in Table 4, and the detailed sequences are provided in the Sequence Listing.

**[Table 3A]**

| Plasmid name | Description | Figure |
|---|---|---|
| Split RNAP plasmids | | |
| pCMV-T7N (d5-19) | N-terminal split T7 RNAP (d5-19) | Fig. 6(a), 9C, 9D |
| pCMV-T7C | C-terminal split T7 RNAP | Fig. 6(a), 9C, , 9D |

| Target-dependent RNAP plasmids | | |
|---|---|---|
| pCMV-T7N-VL (αGCN4) | N-terminal split T7 RNAP (d5-19) -GS linker- VL domain (Anti-GCN4 antibody) | Fig. 1C, 1D, 1E, 2E, 4B, 4D, 5C, 5D, Fig. 6(a), 6(b) |
| pCMV-VH-T7C (αGCN4) | VH domain -GS linker- C-terminal split T7 RNAP (Anti-GCN4 antibody) | Fig. 1C, 1D, 2E, 4B, 5C, 5D, Fig. 6(a), 6(b) |
| pCMV-VH-CGG RNAPc (αGCN4) | VH domain -GS linker- C-terminal split CGG RNAP (Anti-GCN4 antibody) | Fig. 4D |
| pCMV-T7N-VH (αGCN4) | N-terminal split T7 RNAP (d5-19) -GS linker- VH domain (Anti-GCN4 antibody) | Fig. 6(a) |
| pCMV-VL-T7C (αGCN4) | VL domain -GS linker- C-terminal split T7 RNAP (Anti-GCN4 antibody) | Fig. 6(a) |
| pCMV-VH-T7C (αGCN4, WT) | VH domain (WT) -GS linker- C-terminal split T7 RNAP (Anti-GCN4 antibody, omega-grafted framework) | Fig. 1E |
| pCMV-VH-T7C (αGCN4, GLW) | VH variant (GLW) -GS linker- C-terminal split T7 RNAP (Anti-GCN4 antibody, omega-grafted framework) | Fig. 1E |
| pCMV-VH-T7C (αGCN4, ALF) | VH variant (ALF) -GS linker- C-terminal split T7 RNAP (Anti-GCN4 antibody, omega-grafted framework) | Fig. 1E |
| pCMV-VH-T7C (αGCN4, GFA) | VH variant (GFA) -GS linker- C-terminal split T7 RNAP (Anti-GCN4 antibody, omega-grafted framework) | Fig. 1E |
| pCMV-T7N-VL (αFLAG, original) | N-terminal split T7 RNAP (d5-19) -GS linker- VL domain (Anti-FLAG antibody, original framework) | Fig. 2B, Fig. 7(a), 7(b) |
| pCMV-VH-T7C (αFLAG, original) | VH domain -GS linker- C-terminal split T7 RNAP (Anti-FLAG antibody, original framework) | Fig. 2B, Fig. 7(a), 7(b) |
| pCMV-T7N-VL (αFLAG, grafted) | N-terminal split T7 RNAP (d5-19) -GS linker- VL domain (Anti-FLAG antibody, trastuzumab framework) | Fig. 2B, 2C, 2E |
| pCMV-VH-T7C (αFLAG, grafted) | VH domain -GS linker- C-terminal split T7 RNAP (Anti-FLAG antibody, trastuzumab framework) | Fig. 2B, 2C, 2E |
| pCMV-T7N-VL (αEGFP) | N-terminal split T7 RNAP (d5-19) -GS linker- VL domain (Anti-EGFP antibody) | Fig. 2D, 2E, 4D |
| pCMV-VH-T7C (αEGFP) | VH domain -GS linker- C-terminal split T7 RNAP (Anti-EGFP antibody) | Fig. 2D, 2E, 4D |
| pCMV-T7N-VL (αHCV IRES) | N-terminal split T7 RNAP (d5-19) -GS linker- VL domain (Anti-HCV IRES RNA antibody) | Fig. 3B, 3C, Fig. 8(a), 8(b) |
| pCMV-VH-T7C (αHCV IRES) | VH domain -GS linker- C-terminal split T7 RNAP (Anti-HCV IRES RNA antibody) | Fig. 3B, 3C, Fig. 8(a), 8(b) |

**[Table 3B]**

| | | |
|---|---|---|
| pCMV-T7N-VL (αFluorescein) | N-terminal split T7 RNAP (d5-19) -GS linker- VL domain (Anti-Fluorescein antibody) | Fig. 3D, 3E |
| pCMV-VH-T7C (αFluorescein) | VH domain -GS linker- C-terminal split T7 RNAP (Anti-Fluorescein antibody) | Fig. 3D, 3E |
| pCMV-T7N-VL (αHsp70) | N-terminal split T7 RNAP (d5-19) -GS linker- VL domain (Anti-Hsp70 antibody) | Fig. 9C, 9D |
| pCMV-VH-T7C (αHsp70) | VH domain -GS linker- C-terminal split T7 RNAP (Anti-Hsp70 antibody) | Fig. 9C, 9D |

| Induction plasmids | | |
|---|---|---|
| pCMV-EGFP | EGFP | Fig. 1C, 1D, 1E, 2B, 2C, 2D, 2E, 4B, 4D, Fig. 6(a), 6(b), 7(a) |
| pCMV-EGFP-GCN4 | EGFP -GS linker- GCN4 peptide | Fig. 1C, 1D, 1E, 2E, 4B, 4D, Fig. 6(a), 6(b) |
| pCMV-EGFP-1xFLAG | EGFP -GS linker- 1xFLAG peptide | Fig. 2B, 2C, 2E, Fig. 7(a) |
| pCMV-EGFP-3xFLAG | EGFP -GS linker- 3xFLAG peptide | Fig. 2B, 2C, 2E, Fig. 7(a) |
| pCMV-Azami-Green | Azami-Green | Fig. 2D, 2E, 4D |
| pCMV-iRFP670 | iRFP670 | Fig. 4D |
| pCMV-iRFP670-GCN4 | iRFP670 -GS linker- GCN4 peptide | Fig. 4D |
| pFR_HCV_xb | Bi-cistronic construct encoding luciferase (Firefly -HCV IRES- *Renilla*) under HSV TK promoter (Addgene_#11510) | Fig. 3B, 3C, Fig. 8(a), 8(b) |
| pFluc | Mono-cistronic construct encoding firefly luciferase under HSV TK promoter | Fig. 3B, 3C, Fig. 8(a), 8(b) |
| pRluc | Mono-cistronic construct encoding *Renilla* luciferase under HSV TK promoter | Fig. 3B, 3C, Fig. 8(a), 8(b) |

| Reporter plasmids | | |
|---|---|---|
| pT7-IRES2-iRFP670 | EMCV IRES-driven iRFP670 under T7 promoter | Fig. 1C |
| pT7-IRES2-tdTomato | EMCV IRES-driven tdTomato under T7 promoter | Fig. 3B, 3C, 3D, Fig. 7(a), 8(a), 9C, 9D |
| pT7-IRES2-Fluc-pTK-Rluc | Construct encoding EMCV IRES-driven firefly luciferase under T7 promoter and *Renilla* luciferase under HSV TK promoter | Fig. 1D, 1E, 2B, 2C, 2D, 2E, 3E |
| pDual-IRES2-Fluc-pTK-Rluc | Construct encoding EMCV IRES-driven firefly luciferase under both CGG promoter and T7 promoter (CGG pro. - T7 pro. -EMCV IRES- firefly luciferase) and *Renilla* luciferase under HSV TK promoter | Fig. 4B |
| pT7-IRES2-CGG RNAP | EMCV IRES-driven CGG RNAP under T7 promoter | Fig. 4B |
| pT7-IRES2-Fluc | EMCV IRES-driven firefly luciferase under T7 promoter | Fig. 4D |
| pCGG-IRES2-Rluc | EMCV IRES-driven *Renilla* luciferase under CGG promoter | Fig. 4D |
| pT7-gRNA_EGFP | EGFP-targeting gRNA under T7 promoter | Fig. 5C, 5D |

**[Table 3C]**

| Plasmids used for cell line establishment and genome editing | | |
|---|---|---|
| AAV-CMV-EGFP | EGFP gene under CMV promoter is flanked by homology arms targeting human AAVS1 locus. (A splicing acceptor-T2A-PuroR cassette is located downstream of the left homology arm.) | Fig. 5B, 5C, 5D, 5E |
| AAV-CMV-EGFP-GCN4 | EGFP-GCN4 gene under CMV promoter is flanked by homology arms targeting human AAVS1 locus. (A splicing acceptor-T2A-PuroR cassette is located downstream of the left homology arm.) | Fig. 5B, 5C, 5D,5E |
| hCas9 | SpCas9 under CMV promoter (Addgene_##41815) | Fig. 5B, 5C, 5D,5E |
| gRNA_AAVS1-T2 | Human AAVS1 locus-targeting gRNA under human U6 promoter (Addgene_#41818) | Fig. 5B |
| pU6-gRNA_EGFP | EGFP-targeting gRNA under human U6 promoter | Fig. 5E |

| Other plasmids | | |
|---|---|---|
| pHSP70-EGFP | EGFP under human HSP70 promoter | Fig. S4B |
| pUC19 | Empty vector | Fig. 1D, 2C, 2D, 4B, 5C, 5D, 5E, Fig. 8(a) |
| pcDNA3.1 | Empty vector | Fig. 1E, Fig. 6(a) |

**[Table 4]**

| Name of Peptide or Nucleic Acid | Sequence ID No. |
|---|---|
| T7N (d5-19) (N-terminal T7 RNAP fragment) | 12 |
| T7C (C-terminal T7 RNAP fragment) | 13 |
| CGG RNAPc (C-terminal CGG RNAP fragment) | 14 |
| CGG RNAP | 15 |
| VL domain (Anti-GCN4 antibody) | 16 |
| VH domain (Anti-GCN4 antibody) | 17 |
| VH domain (WT), (Anti-GCN4 antibody, omega-grafted framework) | 18 |
| VH domain (GLW), (Anti-GCN4 antibody, omega-grafted framework) | 19 |
| VH domain (ALF), (Anti-GCN4 antibody, omega-grafted framework) | 20 |
| VH domain (GFA), (Anti-GCN4 antibody, omega-grafted framework) | 21 |
| VL domain (Anti-FLAG antibody, trastuzumab framework) | 22 |
| VH domain (Anti-FLAG antibody, trastuzumab framework) | 23 |
| VL domain (Anti-EGFP antibody) | 24 |
| VH domain (Anti-EGFP antibody) | 25 |
| VL domain (Anti-HCV IRES RNA antibody) | 26 |
| VH domain (Anti-HCV IRES RNA antibody) | 27 |
| VL domain (Anti-Fluorescein antibody) | 28 |
| VH domain (Anti-Fluorescein antibody) | 29 |
| VL domain (Anti-Hsp70 antibody) | 30 |
| VH domain (Anti-Hsp70 antibody) | 31 |
| GCN4 peptide | 32 |
| 1xFLAG peptide | 33 |
| 3xFLAG peptide | 34 |
| T7 promoter | 35 |
| CGG promoter | 36 |
| HCV IRES | 37 |
| EGFP-targeting gRNA | 38 |
| EGFP | 39 |
| Human HSP70 promoter | 40 |

### Cell culture and stimulation

293FT cells (Thermo Fisher Scientific) were cultured in DMEM (Nacalai Tesque) supplemented with 10% FBS (Biosera), MEM Non-Essential Amino Acids Solution (Thermo Fisher Scientific), 1 mM sodium pyruvate (Sigma-Aldrich), and 1 mM L-glutamine (Thermo Fisher Scientific) in a humidified incubator at 37°C with 5% CO₂. The cells at < 30 passages were used for all the experiments. The cells at different passages were used for preparing each biological replicate experiment. In the fluorescein induction experiment, the cells were treated with fluorescein diacetate (FUJIFILM Wako Pure Chemical Corporation) dissolved in ethanol at a final concentration of 50 or 100 µg/mL, 16 hours after transfection. The cells were cultured in the media with fluorescein until the subsequent analysis and were washed with PBS before the analysis. In the heat shock experiment, the cells were exposed to 42°C for one hour 16 hours after transfection.

### Plasmid transfection

293FT cells were seeded in 24-well plates at 1 × 10⁵ cells per well the day before transfection. The cells were transfected with a mixture of plasmids using 2.0 µL of Lipofectamine 2000 (Thermo Fisher Scientific). The amounts of transfected plasmids are listed in Tables 5A, 5B, and 5C.

**[Table 5A]**

| Plasmids mixtures for transfection | | | |
|---|---|---|---|
| Figure | Plasmid | Amount (ng) | Total (ng) |
| Fig. 1C | pCMV-T7N-VL (αGCN4) | 15 | 150 |
| | pCMV-VH-T7C (αGCN4) | 35 | |
| | pCMV-EGFP | 0 or 50 | |
| | pCMV-EGFP-GCN4 | 0 or 50 | |
| | pT7-IRES2-iRFP670 | 50 | |
| Fig. 1D | pCMV-T7N-VL (αGCN4) | 15 | 180 |
| | pCMV-VH-T7C (αGCN4) | 35 | |
| | pCMV-EGFP | 0, 10, 20, 40, 60, or 80 | |
| | pCMV-EGFP-GCN4 | 0, 10, 20, 40, 60, or 80 | |
| | pT7-IRES2-Fluc-pTK-Rluc | 50 | |
| | pUC19 | up to 180 | |
| Fig. 1E | pCMV-T7N-VL (αGCN4) | 15 | 150 |
| | pCMV-VH-T7C (αGCN4), (WT, GLW, ALF, or GFA) | 35 | |
| | pCMV-EGFP | 0 or 50 | |
| | pCMV-EGFP-GCN4 | 0 or 50 | |
| | pT7-IRES2-Fluc-pTK-Rluc | 50 | |
| Fig. 2B | pCMV-T7N-VL (αFLAG), (original or grafted) | 25 | 200 |
| | pCMV-VH-T7C (αFLAG), (original or grafted) | 25 | |
| | pCMV-EGFP | 0 or 100 | |
| | pCMV-EGFP-3xFLAG | 0 or 100 | |
| | pT7-IRES2-Fluc-pTK-Rluc | 50 | |
| Fig. 2C | pCMV-T7N-VL (αFLAG), (original or grafted) | 25 | 300 |
| | pCMV-VH-T7C (αFLAG), (original or grafted) | 25 | |
| | pCMV-EGFP | 0, 50, 100, or 200 | |
| | pCMV-EGFP-1xFLAG | 0, 50, 100, or 200 | |
| | pCMV-EGFP-3xFLAG | 0, 50, 100, or 200 | |
| | pT7-IRES2-Fluc-pTK-Rluc | 50 | |
| | pUC19 | up to 300 | |
| Fig. 2D | pCMV-T7N-VL (αEGFP) | 25 | 300 |
| | pCMV-VH-T7C (αEGFP) | 25 | |
| | pCMV-Azami-Green | 0, 50, 100, or 200 | |
| | pCMV-EGFP | 0, 50, 100, or 200 | |
| | pT7-IRES2-Fluc-pTK-Rluc | 50 | |
| | pUC19 | up to 300 | |
| Fig. 2E (left) | pCMV-T7N-VL (αGCN4) | 15 | 200 |
| | pCMV-VH-T7C (αGCN4) | 35 | |
| | pCMV-Azami-Green | 0 or 100 | |
| | pCMV-EGFP | 0 or 100 | |
| | pCMV-EGFP-GCN4 | 0 or 100 | |
| | pCMV-EGFP-3xFLAG | 0 or 100 | |
| | pT7-IRES2-Fluc-pTK-Rluc | 50 | |
| Fig. 2E (middle) | pCMV-T7N-VL (αFLAG, original) | 25 | 200 |
| | pCMV-VH-T7C (αFLAG, original) | 25 | |
| | pCMV-Azami-Green | 0 or 100 | |
| | pCMV-EGFP | 0 or 100 | |
| | pCMV-EGFP-GCN4 | 0 or 100 | |
| | pCMV-EGFP-3xFLAG | 0 or 100 | |
| | pT7-IRES2-Fluc-pTK-Rluc | 50 | |

**[Table 5B]**

| | | | |
|---|---|---|---|
| Fig. 2E (right) | pCMV-T7N-VL (αEGFP) | 25 | 200 |
| | pCMV-VH-T7C (αEGFP) | 25 | |
| | pCMV-Azami-Green | 0 or 100 | |
| | pCMV-EGFP | 0 or 100 | |
| | pCMV-EGFP-GCN4 | 0 or 100 | |
| | pCMV-EGFP-3xFLAG | 0 or 100 | |
| | pT7-IRES2-Fluc-pTK-Rluc | 50 | |
| Fig. 3B (left) | pCMV-T7N-VL (αHCV IRES) | 25 | 300 |
| Fig. 8(a) | pCMV-VH-T7C (αHCV IRES) | 25 | |
| Fig. 8(b) | pFluc | 150 | |
| | pRluc | 50 | |
| | pT7-IRES2-tdTomato | 50 | |
| Fig. 3B (right) | pCMV-T7N-VL (αHCV IRES) | 25 | 300 |
| Fig. 8(a) | pCMV-VH-T7C (αHCV IRES) | 25 | |
| Fig. 8(b) | pFR_HCV IRES xb | 200 | |
| | pT7-IRES2-tdTomato | 50 | |
| Fig. 3D | pCMV-T7N-VL (αFluorescein) | 25 | 100 |
| | pCMV-VH-T7C (αFluorescein) | 25 | |
| | pT7-IRES2-tdTomato | 50 | |
| Fig. 3E | pCMV-T7N-VL (αFluorescein) | 25 | 100 |
| | pCMV-VH-T7C (αFluorescein) | 25 | |
| | pT7-IRES2-Fluc-pTK-Rluc | 50 | |
| Fig. 4B | pCMV-T7N-VL (αGCN4) | 15 | 150 |
| | pCMV-VH-T7C (αGCN4) | 35 | |
| | pCMV-EGFP | 0 or 10 | |
| | pCMV-EGFP-GCN4 | 0 or 10 | |
| | pT7-IRES2-CGG RNAP | 0-40 | |
| | pDual-IRES2-Fluc-pTK-Rluc | 50 | |
| | pUC19 | up to 150 | |
| Fig. 4D | pCMV-T7N-VL (αGCN4) | 12.5 | 400 |
| | pCMV-VH-CGG RNAPc (αGCN4) | 12.5 | |
| | pCMV-T7N-VL (αEGFP) | 50 | |
| | pCMV-VH-T7C (αEGFP) | 50 | |
| | pCMV-Azami-Green | 0 or 100 | |
| | pCMV-EGFP | 0 or 100 | |
| | pCMV-iRFP670 | 0 or 25 | |
| | pCMV-iRFP670-GCN4 | 0 or 25 | |
| | pT7-IRES2-Fluc | 50 | |
| | pCGG-IRES2-Rluc | 100 | |
| Fig. 5B | AAV-CMV-EGFP | 0 or 500 | 1500 |
| | AAV-CMV-EGFP-GCN4 | 0 or 500 | |
| | hCas9 | 500 | |
| | gRNA AAVS1-T2 | 500 | |
| Fig. 5C (top) | pCMV-T7N-VL (αGCN4) | 15 | 350 |
| Fig. 5D | pCMV-VH-T7C (αGCN4) | 35 | |
| | hCas9 | 200 | |
| | pT7-gRNA EGFP | 0 or 100 | |
| | pUC19 | 0 or 100 | |
| Fig. 5C (bottom) | pCMV-T7N-VL (αGCN4) | 15 | 550 |
| | pCMV-VH-T7C (αGCN4) | 35 | |
| | hCas9 | 400 | |
| | pT7-gRNA_EGFP | 0 or 100 | |
| | pUC19 | 0 or 100 | |
| Fig. 5E | pCMV-T7N-VL (αGCN4) | 15 | 350 |
| | pCMV-VH-T7C (αGCN4) | 35 | |
| | hCas9 | 200 | |
| | pU6-gRNA_EGFP | 0 or 100 | |
| | pUC19 | 0 or 100 | |

**[Table 5C]**

| | | | |
|---|---|---|---|
| Fig. 1E (RNAP-) | pcDNA3.1 | 50 | 150 |
| Fig. 6(a) (RNAP-) | pCMV-EGFP | 0 or 50 | |
| | pCMV-EGFP-GCN4 | 0 or 50 | |
| | pT7-IRES2-Fluc-pTK-Rluc | 50 | |
| Fig. 6(a) (None) | pCMV-T7N (d5-19) | 15 | 150 |
| | pCMV-T7C | 35 | |
| | pCMV-EGFP | 0 or 50 | |
| | pCMV-EGFP-GCN4 | 0 or 50 | |
| | pT7-IRES2-Fluc-pTK-Rluc | 50 | |
| Fig. 6(a) (VH_VL) | pCMV-T7N-VH (αGCN4) | 15 | 150 |
| | pCMV-VL-T7C (αGCN4) | 35 | |
| | pCMV-EGFP | 0 or 50 | |
| | pCMV-EGFP-GCN4 | 0 or 50 | |
| | pT7-IRES2-Fluc-pTK-Rluc | 50 | |
| Fig. 6(a) (VL_VH) | pCMV-T7N-VL (αGCN4) | 15 | 150 |
| | pCMV-VH-T7C (αGCN4) | 35 | |
| | pCMV-EGFP | 0 or 50 | |
| | pCMV-EGFP-GCN4 | 0 or 50 | |
| | pT7-IRES2-Fluc-pTK-Rluc | 50 | |
| Fig. 6(b) | pCMV-T7N-VL (αGCN4) (1x, 2x, or 3x GS linker) | 15 | 150 |
| | pCMV-VH-T7C (αGCN4) (1x, 2x, or 3x GS linker) | 35 | |
| | pCMV-EGFP | 0 or 50 | |
| | pCMV-EGFP-GCN4 | 0 or 50 | |
| | pT7-IRES2-Fluc-pTK-Rluc | 50 | |
| Fig. 7(a) | pCMV-T7N-VL (αFLAG), (original or grafted) | 25 | 200 |
| | pCMV-VH-T7C (αFLAG), (original or grafted) | 25 | |
| | pCMV-EGFP | 0 or 100 | |
| | pCMV-EGFP-3xFLAG | 0 or 100 | |
| | pT7-IRES2-tdTomato | 50 | |
| Fig. 8(a) (control) | pCMV-T7N-VL (αHCV IRES) | 25 | 300 |
| | pCMV-VH-T7C (αHCV IRES) | 25 | |
| | pUC19 | 200 | |
| | pT7-IRES2-tdTomato | 50 | |
| Fig. 9B | pHSP70-EGFP | 100 | 100 |
| Fig. 9C | pCMV-T7N-VL (αHsp70) | 0 or 100 | 250 |
| Fig. 9D | pCMV-VH-T7C (αHsp70) | 0 or 100 | |
| | pCMV-T7N (d5-19) | 0 or 100 | |
| | pCMV-T7C | 0 or 100 | |
| | pT7-IRES2-tdTomato | 50 | |

### Generation of stable cell lines using CRISPR-Cas9 system

The stable cell lines expressing EGFP-GCN4 or EGFP were generated using the CRISPR-Cas9 system. Each gene was cloned into a donor plasmid with homologous arms targeting the endogenous AAVS1 locus on the genome. 293FT cells were seeded in 24-well plates at 1 × 10⁵ cells per well the day before transfection. The cells were transfected with 1 µg of SpCas9 plasmid (Addgene plasmid #41815), 1 µg of AAVS1-targeting sgRNA plasmid (Addgene plasmid #41818), and 1 µg of the donor plasmid using 2.0 µL of Lipofectamine 2000 (Thermo Fisher Scientific) following the manufacturer's instructions. Two days after the transfection, the cells were trypsinized and seeded into six-well plates with the medium containing 1 µg/mL puromycin (Invivogen). The fluorescence-positive cells were then sorted and enriched using FACSymphony S6 cell sorter (BD Biosciences). The sorted cells were maintained in the medium containing 0.5 µg/mL puromycin.

### Luciferase assay

All the luciferase assays were conducted using Dual-Glo Luciferase Assay System (Promega) following the manufacturer's instructions. 293FT cells were harvested and lysed 2 days after transfection. The activities of firefly luciferase and *Renilla* luciferase were measured using GloMax Navigator Microplate Luminometer (Promega). Relative luciferase units (RLU) were calculated by normalizing firefly luciferase activity to *Renilla* luciferase activity in each sample. Each biological replicate experiment contained two technical replicates.

### Fluorescent reporter assay

The cells were imaged 2 days after transfection using CellVoyager CQ1 (Yokogawa Electric Corporation). Each image was processed using ImageJ software (National Institutes of Health) and its plugin (https://github.com/yfujita-skgcat/image_converter). For quantification, the total integrated intensity of the tdTomato fluorescence-positive cells in each image was quantified as fluorescence intensity using ImageJ. To calculate the mean tdTomato fluorescence intensity, the cells were analyzed using CytoFLEX S Flow Cytometer (Beckman Coulter), and the acquired data were analyzed using FlowJo software (BD Biosciences).

### CRISPR-Cas9-mediated gene knockout

In the CRISPR-Cas9 knockout experiments, the stable cell lines; EGFP-GCN4 cells and EGFP cells, were used. The cells were seeded in 24-well plates and transfected with the plasmids listed in Supplementary Table 4. Three days after the transfection, the cells were trypsinized and seeded into 6 well plates with the medium containing 0.5 µg/mL puromycin. Seven days after the transfection, the cells were analyzed using CytoFLEX S Flow Cytometer. The acquired data were analyzed to calculate the EGFP-negative population using FlowJo software and the "flowCore" package of R (https://bioconductor.org/packages/release/bioc/html/flowCore.html).

### In silico protein structure prediction

The structure of the CDR loop-grafted variable domain of the anti-FLAG antibody was predicted using ColabFold, a Google Colab-based protein structure prediction with AlphaFold2 and MMseqs2, with default settings. The predicted structures were visualized and aligned to the original variable region of the anti-FLAG antibody (PDB ID: 7BG1) using PyMOL software.

### [Results]

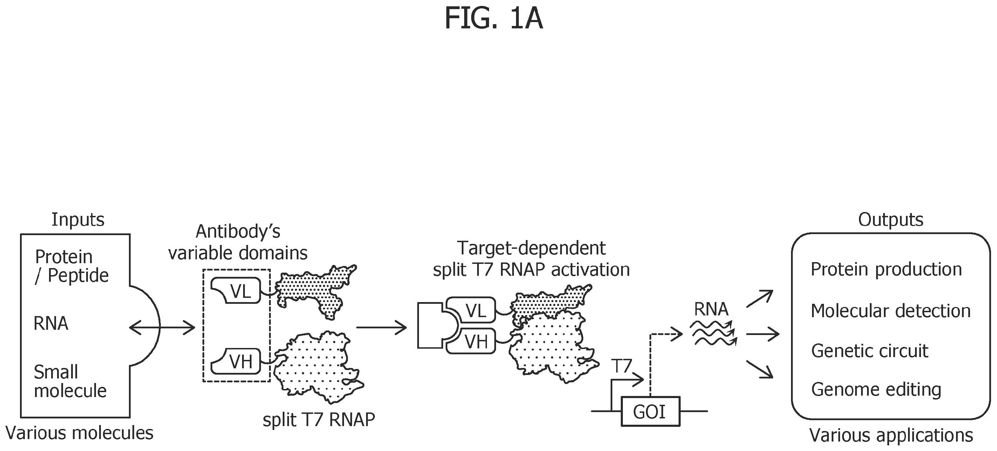

To regulate gene expression in response to various intracellular molecules, we conceived the idea of using the variable domain of an antibody as a fusion protein with split RNAP. In realizing this concept, we aimed to develop a system that assembles split RNAP based on target binding by variable domains of single antibody, rather than using two antibodies. This is because it allows regulating gene expression using identified antibodies without the screening of secondary antibodies for split RNAP assembly. To enable this strategy, we focused on the structure of the variable domain. The variable domain consists of heavy and light chains and thus can be divided into two small domains: heavy-chain variable domain and light-chain variable domain (VH and VL). Therefore, we hypothesized that VH and VL domains could induce the assembly of split RNAP by binding to their target molecules (FIG. 1A). We named this system as "Target-dependent RNAP (TdRNAP)".

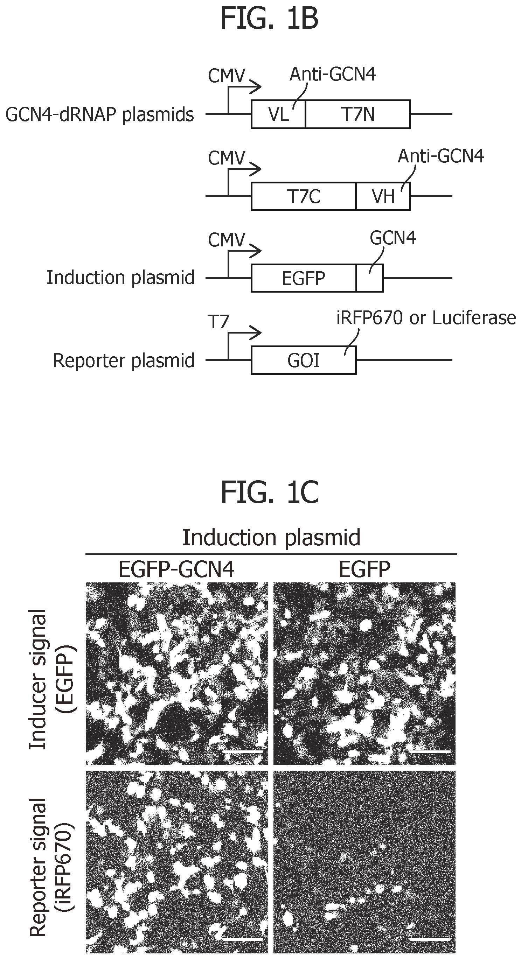

To test our hypothesis, we first designed a TdRNAP using the VH and VL domains of an anti-GCN4 antibody. The anti-GCN4 antibody recognizes a leucine zipper peptide of the yeast transcription factor GCN4 with nanomolar affinity, and its variable domain has an optimized framework region that allows protein folding without relying on intramolecular disulfide bonds, which ensures the structural stability in the intracellular reducing environment. We fused the VH and VL domains to C-terminal and N-terminal fragments of split T7 RNAP, respectively, resulting in GCN4-dependent RNAP (GCN4-dRNAP) (FIG. 1B). To test the GCN4-dRNAP, we used enhanced green fluorescent protein (EGFP) fused with GCN4 peptide (EGFP-GCN4) as a target molecule and investigated whether the induction with EGFP-GCN4 induces split RNAP assembly. The expression plasmid for GCN4-dRNAP were co-transfected into the human embryonic kidney 293FT cells along with a reporter plasmid encoding near-infrared fluorescent protein 670 (iRFP670) under the control of T7 promoter, and an induction plasmid encoding EGFP-GCN4 or EGFP. We then analyzed iRFP670 expression with fluorescent microscopy. The coexpression with EGFP-GCN4 resulted in a strong enhancement in iRFP670 fluorescence. From this result, it was demonstrated that GCN4 binding to VH and VL domains can induce split RNAP assembly (FIG. 1C). This GCN4-dependent reporter expression was observed even when the fusion orientation of VH and VL domains was reversed. The additional reporter assay with luciferase as the reporter gene showed that the GCN4-dRNAP exhibits slightly higher activity when the VH and VL domains are fused to the C-terminal and N-terminal RNAPs, respectively (FIG. 6(a)).

We thus used this fusion pattern (T7N-VL and VH-T7C) for subsequent experiments. We also assessed the effect of linker length on the activity of GCN4 dRNAP. Luciferase assay revealed minimal linker-length dependency in the activity of GCN4-dRNAP (FIG. 6(b)). We noted that T7 RNAP undergoes large conformational changes during the transition from transcription initiation to elongation reaction. This is because larger or highly charged target molecules may interfere with such conformational changes. We decided to use the longer linker for subsequent experiments to minimize such negative effects.

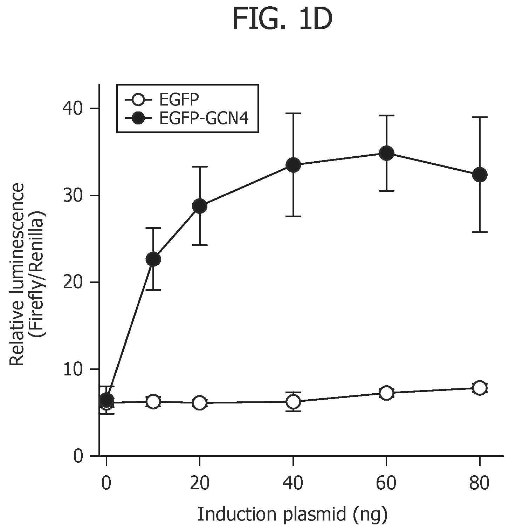

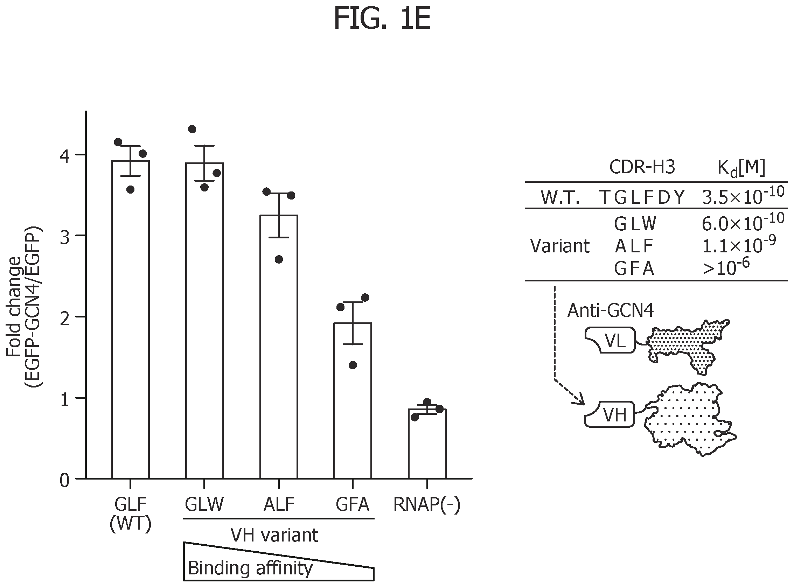

Next, we investigated whether the activity of GCN4-dRNAP increases in a dose-dependent manner with respect to the target molecule. To examine the dose dependency, we varied the amount of EGFP-GCN4 added and conducted luciferase assays. The induction with EGFP-GCN4 resulted in a dose dependent increase in reporter signals, whereas EGFP without GCN4 did not increase the reporter signals even at high concentrations (FIG. 1D). Finally, we investigated whether the binding affinity between the antibody and the target molecule affects the activity of TdRNAP. To examine this affinity dependency, we used three VH variants of the anti-GCN4 antibody with amino acid mutations in the CDR loop which decreased the binding affinity for the GCN4 peptide. The luciferase assay showed the reduction in binding affinity decreases the RNAP activity (FIG. 1E). In particular, GFA, a VH variant with more than 300-fold lower affinity than WT, substantially decreased the RNAP activity. In contrast, GLW, a VH variant with 1.7-fold lower affinity than WT, exhibited comparable activity to WT, suggesting that the dissociation constant of 0.6 nM is strong enough to maximize the activity of TdRNAP in mammalian cells. These results show that the activity of TdRNAP depends on the dose of intracellular target molecules and the binding affinity. Taken together, our results demonstrate that the interaction between VH/VL domains and their target molecule can lead to the assembly of split RNAP and induce target-dependent gene transcription.

### Expanding the repertoire of target molecules by antibody substitution

Next, we investigated whether antibody substitutions allow TdRNAP to induce reporter expression in response to the corresponding target molecules.

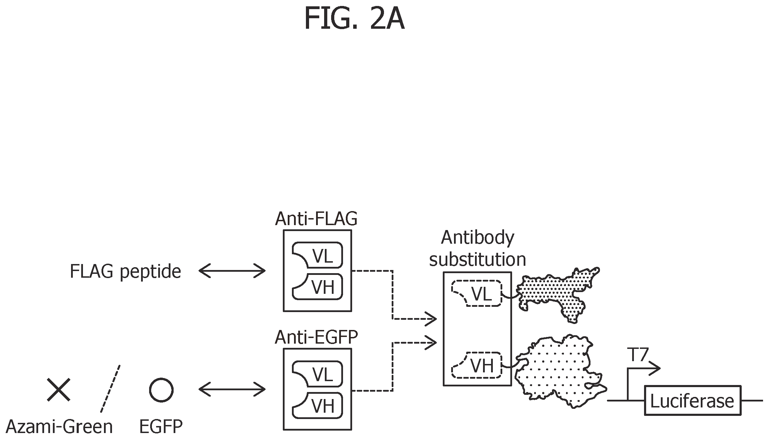

To test this, we first used the VH and VL domains of an anti-FLAG antibody to design FLAG-dependent RNAP (FLAG-dRNAP) (FIG. 2A, top). The anti-FLAG antibody, clone M2, recognizes a FLAG octapeptide (DYKDDDDK) with nanomolar affinity (Kd = 6.5 nM). However, the intracellular stability of its variable domain remains unclear. Upon the induction with FLAG-tagged EGFP (EGFP-1xFLAG), we found that the FLAG-dRNAP could induce reporter expression, but the RNAP activity was very low despite its high binding affinity (FIG. 7(a)). The RNAP activity was not significantly improved even when using three tandem FLAG tags (3xFLAG) which enhance the binding affinity (FIG. 7(a)). From these results, we hypothesized that the structure of this variable domain might be unstable in the reducing environment of the cytoplasm due to its dependence on intramolecular disulfide bonds for proper folding.

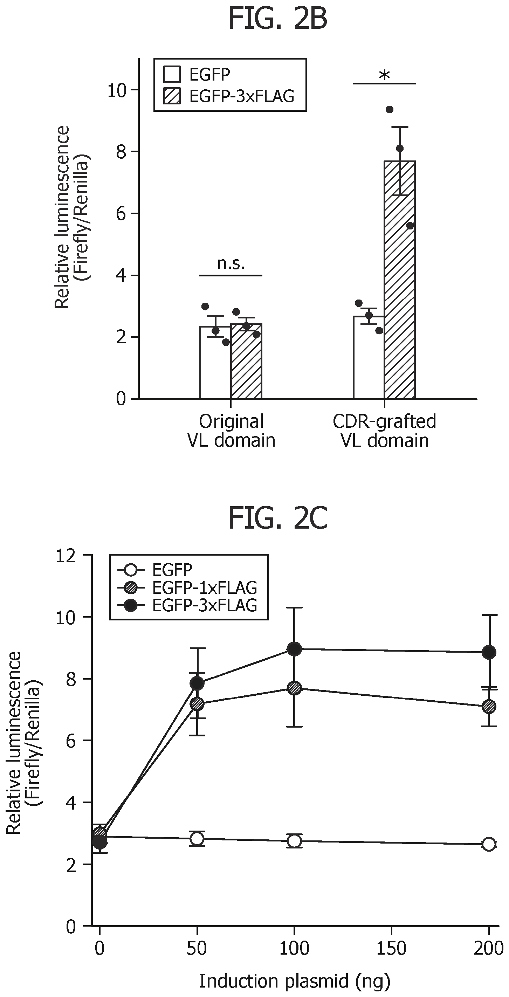

For the purpose of structural stabilization, we grafted the CDR loop of the anti-FLAG antibody onto a stable framework region derived from a mutated humanized trastuzumab antibody, which is an anti-HER2 antibody (structural diagram omitted). As a result, we found that the CDR loop grafting of the VL domain successfully enhanced the activity of FLAG-dRNAP. This result indicates that the structure of the VL domain of the anti-FLAG antibody is unstable within the cells (FIG. 2B). The CDR loop grafting of both VH and VL domains further increased reporter expression, but it also induced leak expression in the absence of the FLAG tag, suggesting that the grafted VH and VL domains of the anti-FLAG antibody weakly associated with each other (FIG. 7(b)). We thus used, for subsequent experiments, the FLAG-dRNAP in which the CDR loop sequences of both the VH and VL domains were derived from the FLAG antibody, the framework region of the VL domain carried the sequence originating from the mutated humanized trastuzumab, and the framework region of the VH domain retained the sequence derived from the original FLAG antibody. Subsequently, we investigated whether this FLAG-dRNAP shows an increase in RNAP activity dependent on concentration and affinity. Upon the induction with EGFP-1xFLAG, we observed a dose-dependent increase in reporter signals (FIG. 2C). The reporter signals were further enhanced upon the induction with EGFP-3xFLAG, indicating that the RNAP activity is affinity-dependent. These observations are consistent with the results obtained with GCN4-dRNAP (FIGs. 1D and 1E). These results demonstrated that antibody substitution allows TdRNAPs to alter their target molecules. Additionally, even if the variable domains of antibodies were unstable within the cells, we showed that the variable domains can be stabilized and adapted for TdRNAP using stable framework regions.

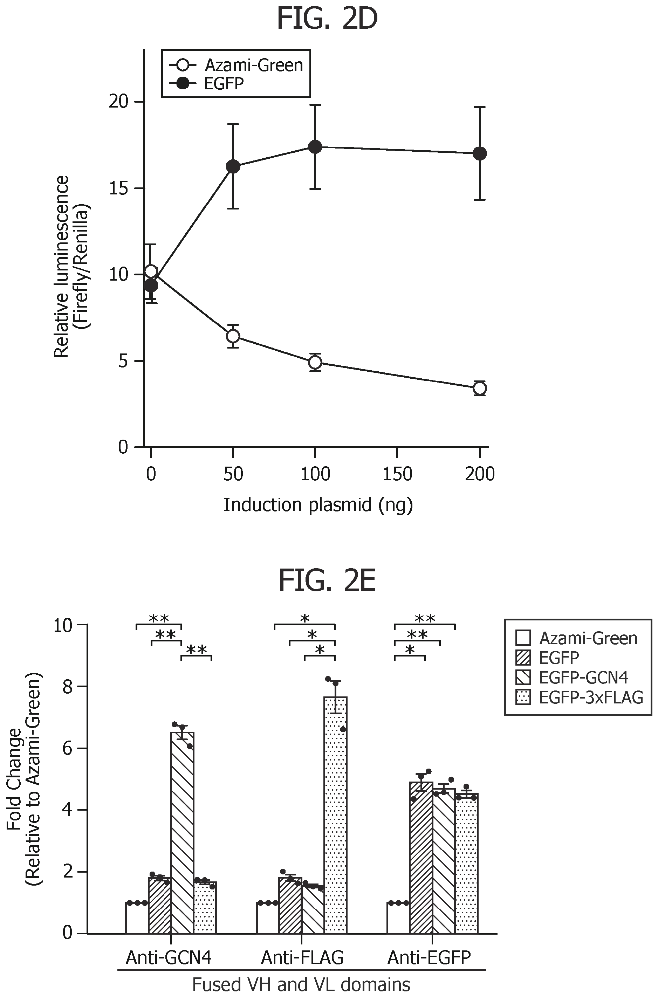

Based on the insight from FLAG-dRNAP, we designed an additional protein-dependent RNAP targeting larger proteins rather than small peptides. We selected an EGFP antibody for the next substitution because the anti-EGFP antibody had been designed based on the trastuzumab stable framework (FIG. 2A, bottom). To examine the target specificity of EGFP-dependent RNAP (EGFP-dRNAP), we used EGFP and Azami-Green as target molecules. Azami-Green is a green fluorescent protein that has a similar β-barrel structure to EGFP but a low sequence identity of 29%. The induction with EGFP successfully increased the reporter signals in a dose-dependent manner, whereas the induction with Azami-Green did not increase the reporter signals even at high concentrations. (FIG. 2D). These results clearly show that EGFP-dRNAP activates through the specific recognition of EGFP.

In addition, we tested whether each TdRNAP could specifically respond to their corresponding targets without cross-reactivity. To examine the cross-reactivity, we used GCN4-, FLAG-, and EGFP-dRNAP, and coexpressed them with individual targets. The results of luciferase assay showed that each TdRNAP was activated only in response to the corresponding target (FIG. 2E), demonstrating that the fused antibody variable domains specifically recognize their corresponding target molecules in mammalian cells. These results show that each VH and VL domain retains its target specificity in mammalian cells, indicating that the CDR loop structures of the antibody variable domains are properly folded and stabilized. This suggests that the stable framework domain and the molecular chaperone of mammalian cells may contribute to the stabilized CDR loop structures and target specificity. Taken together, these results demonstrate that antibody substitution enables the expansion of intracellular targets for TdRNAP, and the substituted antibodies retain the high specificity to the corresponding targets.

### Design of RNA and small-molecule-dependent RNAPs

Our results raised the question of whether TdRNAP could regulate reporter expression in response to other types of molecules, such as RNA and small molecules. Several RNA sensing technologies have already been developed based on engineered RNAs. However, these technologies rely on complementary base pairing for RNA targeting, making it challenging to access complex structured RNAs where target sequences are masked by RNA secondary and tertiary structures. In contrast, antibodies exhibit structure specificity for RNA recognition rather than nucleotide sequence specificity. This structural specificity could provide an advantage in targeting virus RNA when targeting viral RNA because viruses have high mutation rates but maintain conserved structured regions within their genome, which is essential for viral replication and packaging the genomic RNA. We thus aimed to design an RNA-dependent RNAP (RNA-dRNAP) using an anti-viral RNA antibody and sought to investigate whether RNA-dRNAP can detect structured regions of viral RNA within mammalian cells.

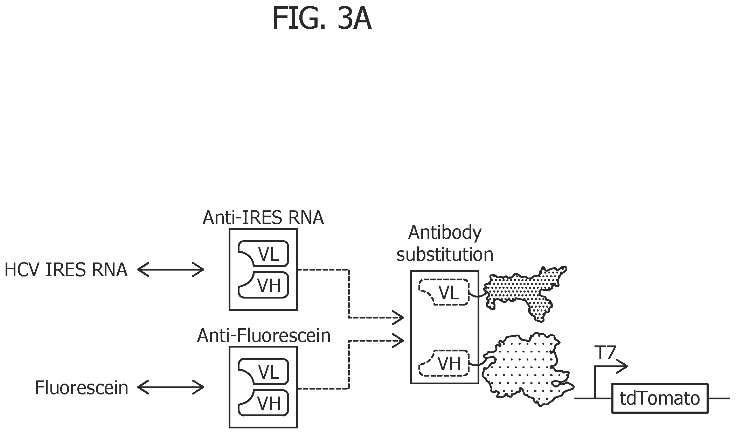

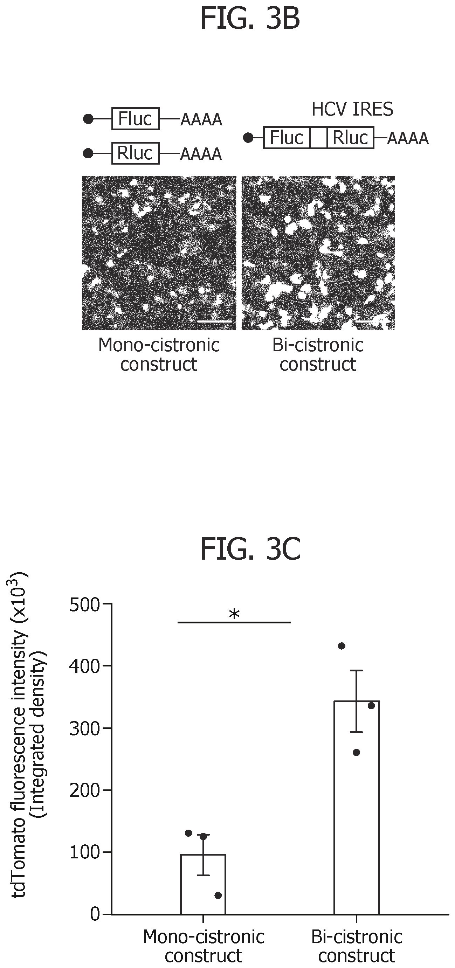

To test this, we used an anti-HCV IRES RNA antibody that specifically recognizes a structured RNA region within the internal ribosome entry site (IRES) of the hepatitis C virus (HCV) genomic RNA. The VH and VL domains, in which the CDR loops of the VH and VL domains of the anti-HCV IRES RNA antibody were grafted to the frameworks derived from mutated humanized trastuzumab, were fused to split RNAP, resulting in IRES RNA-dRNAP (FIG. 3A, top). At that time, we used a VH domain derived from the mutated humanized trastuzumab (Seq ID No. 2) in which the 13th residue Arg in FR3 was substituted with Ala. This was because structural prediction by AlphaFold2 suggested that the 13th Arg in FR3 disrupts the configuration of the CDR loop. In the VH domain of the humanized trastuzumab (Seq ID No. 42), the 13th residue in FR3 is Ala. The expression plasmid of IRES RNA-dRNAP was cotransfected along with a tdTomato reporter plasmid and a bi-cistronic construct with HCV IRES encoding firefly and *Renilla* luciferases. We then observed tdTomato fluorescence. Interestingly, we observed the robust enhancement of tdTomato fluorescence upon the induction with IRES-containing bi-cistronic mRNA (FIGs. 3B, 3C, and 8(a)), whereas the induction with mono-cistronic mRNAs without HCV IRES exhibited lower tdTomato fluorescence. We simultaneously conducted luciferase assays and confirmed the *Renilla* luciferase expression from HCV IRES of the bi-cistronic mRNA, which demonstrates that HCV IRES properly folds into a functional structure in living cells. The luciferase assays also showed that the expression levels of firefly and *Renilla* luciferases were comparable between the bi-cistronic and mono-cistronic mRNAs, indicating that translated luciferases did not affect the RNAP activity (FIG. 8(b)). These results demonstrate that the IRES RNA-dRNAP activates transcription through the detection of the IRES region of HCV in living cells.

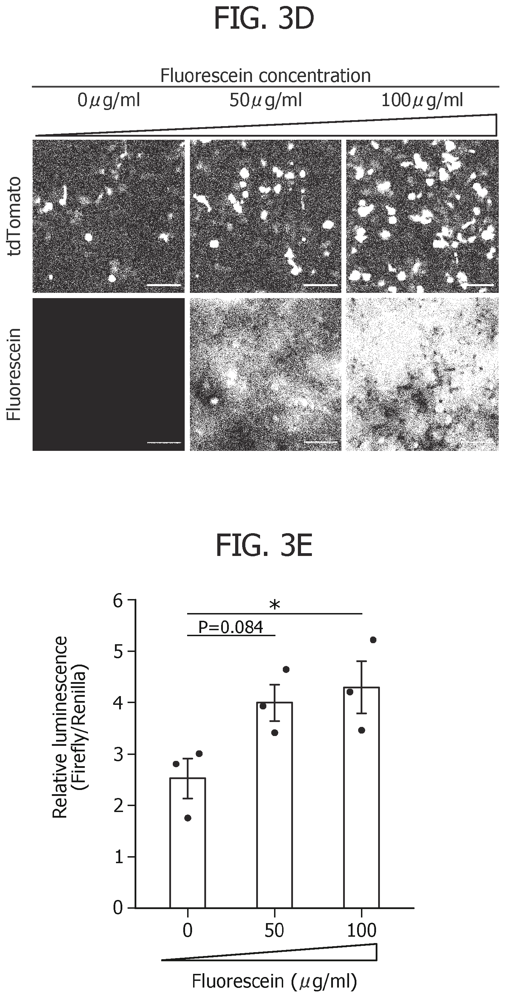

We next investigated whether TdRNAP could respond to small molecules in living cells using anti-small-molecule antibodies. To test this, we employed an anti-fluorescein antibody. We designed a fluorescein-dependent RNAP (Fluorescein-dRNAP) by fusing the VH and VL domains, in which the CDR loops of heavy and light chains of an anti-fluorescein antibody were grafted to the frameworks derived from mutated humanized trastuzumab, to split RNAP (FIG. 3A, bottom). The expression plasmid of IRES RNA-dRNAP was transfected into cells along with the tdTomato reporter plasmid. We then treated the transfected cells with fluorescein diacetate, which is converted into fluorescein within living cells, and performed fluorescent reporter assays. Treatment with fluorescein led to a dose-dependent increase in reporter expression, while the treatment with the vehicle only mildly induced reporter expression (FIGs. 3D and 3E). These results show that fluorescein promotes the assembly of the VH and VL domains and the activation of split RNAP. The results also indicate that the VH and VL domains exhibited a weak association even in the absence of fluorescein. The VH and VL domains of the anti-fluorescein antibody contain several residues in their CDR loops that interact with each other to form a binding pocket for fluorescein loading. This interaction might induce the spontaneous assembly of split RNAP even without fluorescein.

In summary, anti-RNA and anti-small-molecule antibodies can also be adapted for use with TdRNAP. Thus, TdRNAP can serve as a versatile platform for regulating gene expression in response to a broad spectrum of biochemical molecules within mammalian cells.

### Constructing multilayer genetic circuits with TdRNAP

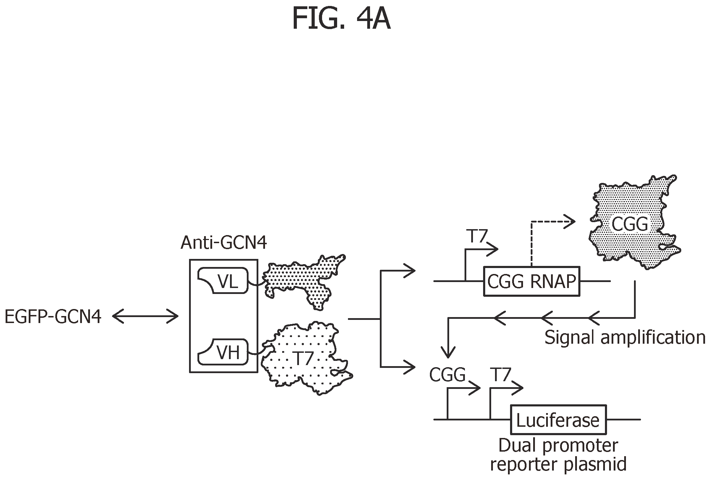

Our results showed that TdRNAP can serve as biochemical information converters that transduce various inputs into desired genetic outputs. With this function, TdRNAP could provide versatile tools for constructing synthetic genetic circuits in living cells. We next sought to apply TdRNAP to develop multilayer genetic circuits that can simultaneously regulate multiple gene expressions depending on the corresponding targets. We first designed a transcriptional amplification system using a T7 RNAP variant, CGG-R12-KIRV (CGG RNAP), as an additional reporter gene. CGG RNAP is an evolved T7 RNAP with amino acid mutations in the C-terminal region that allow RNAP to specifically recognize a mutated T7 promoter, called CGG promoter. We hypothesized that by placing CGG RNAP under the control of the T7 promoter and adding the CGG promoter to existing T7 promoter-driven reporter plasmids, CGG RNAP could be used to amplify reporter gene expression (Figure 4A). This transcriptional amplification system is considered advantageous for detecting the low amount of intracellular molecules. We thus inserted CGG promoter upstream of T7 promoter, resulting in a dual promoter reporter plasmid expressing luciferase under the control of T7 and CGG promoters.

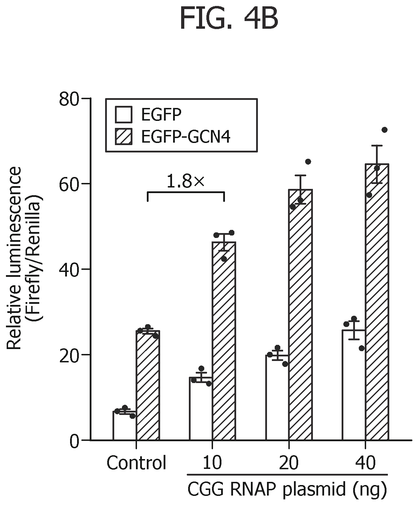

To test this transcriptional amplification system, we used GCN4-dRNAP and examined whether the reporter signals were enhanced even at low amounts of EGFP-GCN4. The expression plasmid for GCN4-dRNAP were transfected into 293FT cells along with the dual promoter reporter plasmid and the additional reporter plasmid encoding CGG RNAP. Upon the induction with 10 ng of the EGFP-GCN4 expression plasmid, the transcriptional amplification system exhibited a 1.8-fold enhancement of reporter expression compared with the non-amplification system (FIG. 4B). The amplified signal was 1.3-fold higher than the saturated value of the non-amplification system which was observed using six times more of the EGFP-GCN4 expression plasmid (60 ng) (FIGs. 1D and 4B). The transcriptional amplification system further enhanced the reporter signals depending on the amount of T7 promoter-driven CGG RNAP plasmid, although the background levels were also increased. These observations show that the additional reporter layer of CGG RNAP plays the role of a signal amplifier and is able to amplify the reporter signals even at low concentrations of the target molecule.

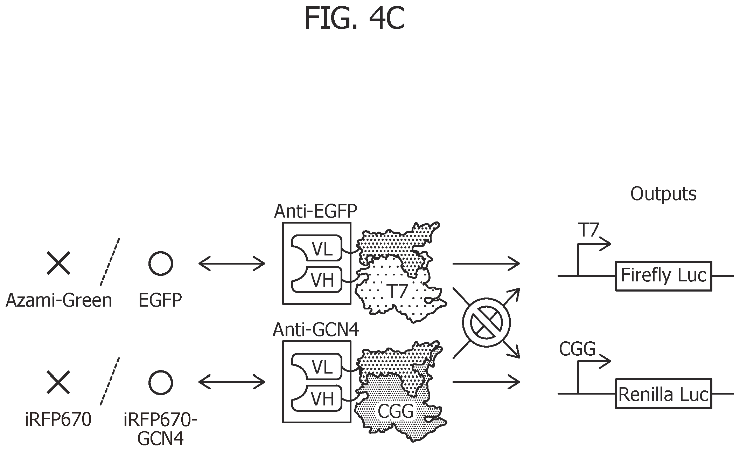

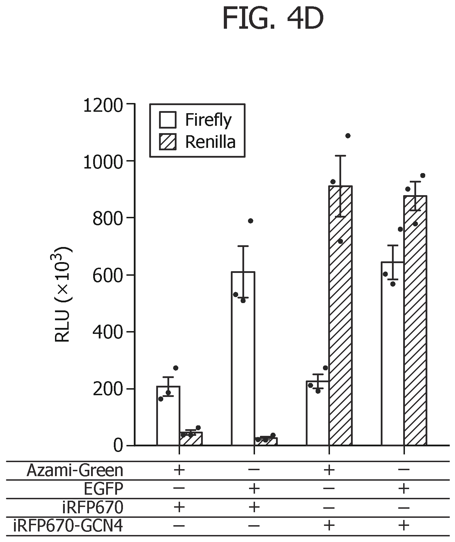

Next, we designed an orthogonal genetic circuit using two TdRNAPs that can simultaneously respond to two different targets and independently regulate two reporter genes. As a proof of concept, we designed the orthogonal system consisting of EGFP-dRNAP and GCN4-dRNAP. To independently regulate two reporter genes, EGFP-dRNAP and GCN4-dRNAP were constructed with split T7 RNAP and split CGG RNAP, respectively, resulting in EGFP-dependent T7 RNAP and GCN4-dependent CGG RNAP (FIG. 4C). We investigated whether these two TdRNAPs could exhibit pattern of orthogonal gene expression in a target dependent manner using firefly and *Renilla* luciferase genes under the control of T7 and CGG promoters, respectively. The luciferase assay showed that the orthogonal system precisely regulates the expressions of two reporter genes depending on the presence pattern of the corresponding target molecules (FIG. 4D). This result demonstrates that two TdRNAPs simultaneously regulate their respective reporter genes in parallel. Taken together, these results show that the combination of antibodies and T7 RNAP variants expands the pattern of output signals and provides versatile tools for constructing multilayer genetic circuits in living cells.

### Target-dependent genome editing in human cells

The regulation of genome editing between targeted and non-targeted cells is one of the major challenges in gene therapy. To achieve such cell-specific genome editing, several biomarkers, including cell surface proteins and microRNAs, have been utilized to regulate the delivery and expression of genome editing machinery. However, many of these biomarkers are by-products with elevated expression levels during disease progression, and the available biomarkers for the conventional approaches are still limited. Ideally, genome editing should be autonomously driven by detecting gene products derived from target genes with genetic mutations. In this strategy, TdRNAP has the advantage of intracellular target selectivity to regulate gene expression. Moreover, TdRNAP can generate functional RNA, such as guide RNA (gRNA) for genome editing. We thus hypothesized that TdRNAP could autonomously trigger genome editing by directly recognizing disease-associated proteins, such as mutant and fusion proteins, expressed from target genes in the genome.

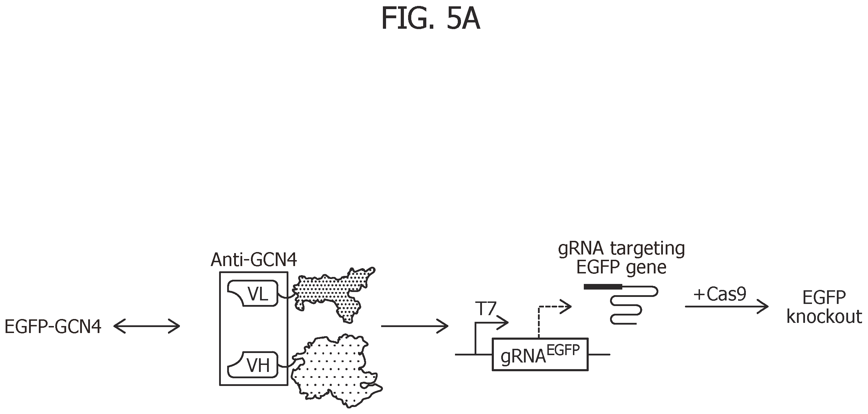

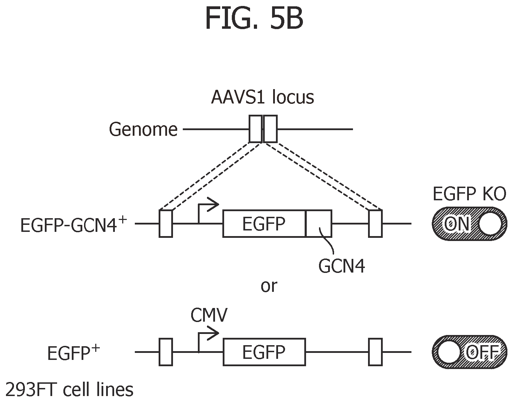

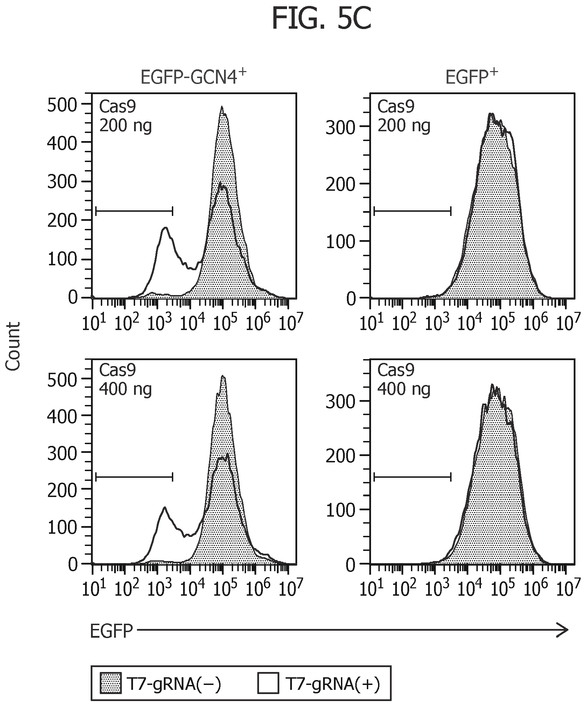

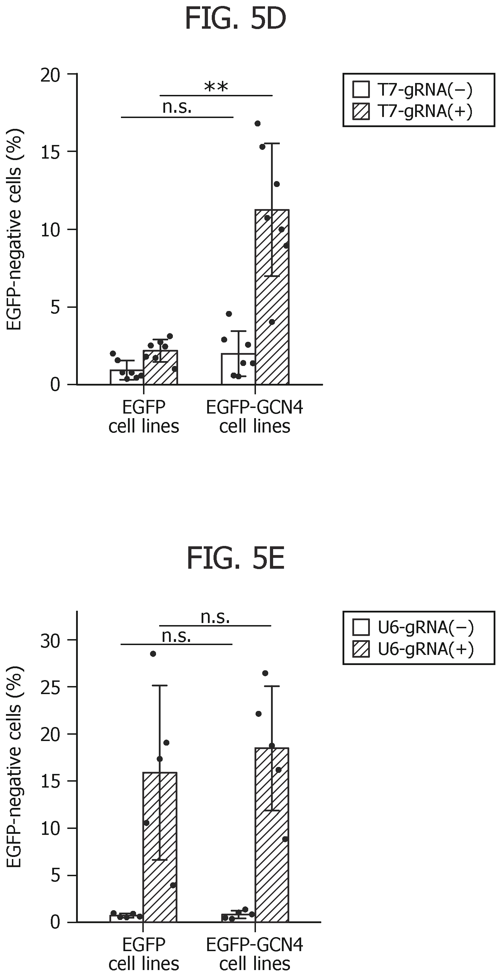

To prove this concept, we designed a gene knockout experiment using a CRISPR-Cas9 system. In the system, DNA cleavage was regulated by TdRNAP and preferentially induced in cells expressing a fusion gene (FIG. 5A). We aimed to induce EGFP knockout in a GCN4-dependent manner using GCN4-dRNAP and an EGFP-targeting gRNA under the control of T7 promoter. To test this, we used EGFP-GCN4 and EGFP genes as fusion and intact genes, respectively, and established two 293FT cell lines carrying EGFP-GCN4 or EGFP gene in the AAVS1 locus on the genome (FIG. 5B). The constructs for GCN4-dRNAP and plasmid for gRNA expression were cotransfected into EGFP-GCN4 and EGFP cell lines together with a Cas9 expression plasmid. We then measured the EGFP-negative cell population by flow cytometry. Flow cytometry analysis showed that GCN4-dRNAP induced EGFP knockout more preferentially in EGFP-GCN4 cell lines than EGFP cell lines, demonstrating that EGFP-GCN4 itself was used as a driver to knockout its own gene (FIGs. 5C and 5D). Notably, we did not observe the increase in EGFP-negative cell population in EGFP cell lines even at high Cas9 expression levels (FIG. 5C). These results indicate that the EGFP knockout event is tightly regulated by GCN4-dRNAP through regulating gRNA expression depending on intracellular EGFP-GCN4. For comparison, we used a constitutive gRNA expression plasmid with U6 promoter and conducted the same knockout experiment in which gRNA expression is independent of GCN4. The constitutive gRNA expression resulted in EGFP knockout with comparable efficiency between two cell lines, indicating that the insertion of the gene sequence for GCN4 peptide does not affect the knockout efficiency (FIG. 5E). These results demonstrate that TdRNAP can autonomously trigger gRNA expression by direct recognition of aberrant proteins expressed from target genes in the genome, enabling precise genome editing in a cell-specific manner.

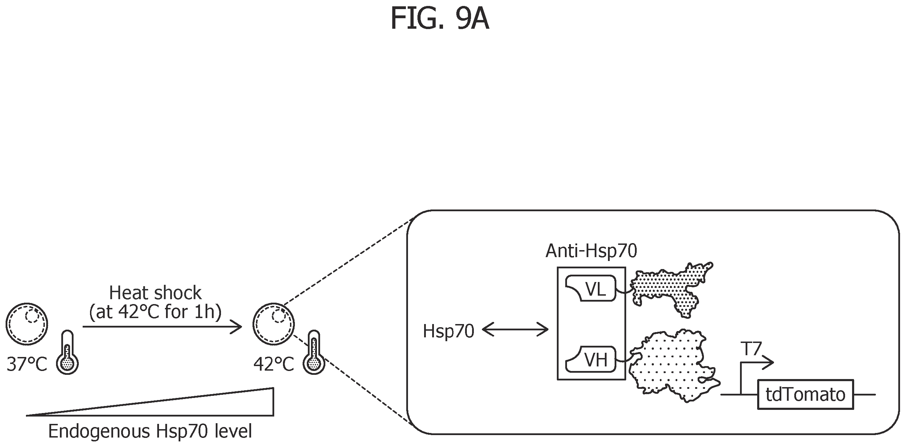

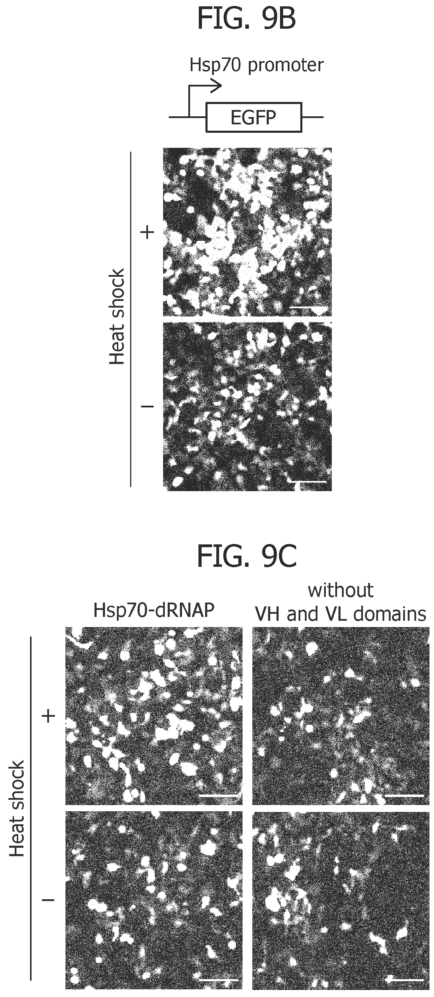

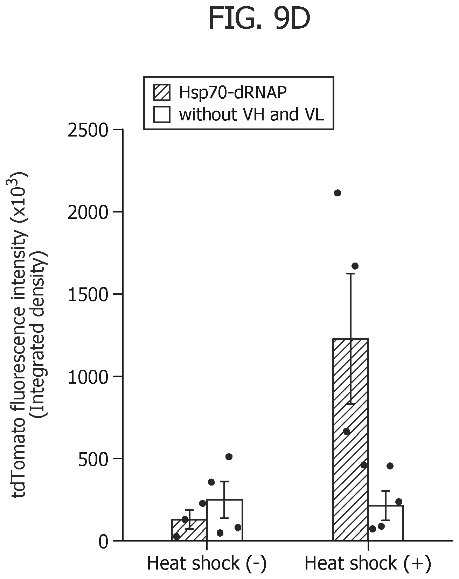

We demonstrated that TdRNAP induces genome editing in response to gene products expressed from a single genomic locus, indicating that TdRNAP is sufficiently sensitive to intracellular gene products expressed at more moderate and uniform levels, rather than transient overexpression resulting from plasmid transfection. Furthermore, TdRNAP engineered using an anti-Hsp70 antibody was found to respond to the increase in endogenous Hsp70 expression following heat-shock stimulation (FIG. 9A, 9B, 9C, and 9D). These findings suggest that TdRNAP can regulate transcriptional activity in a manner dependent on the expression level of endogenous proteins. Such dose-dependent activity is useful for targeting cancer cells with abnormal gene expression without affecting healthy cells. The ability to regulate gene expression may open new possibilities for personalized medicine and precision therapies. TdRNAP represents a robust and versatile strategy for engineering the regulation of gene circuits and cellular functions, and is expected to be applicable to both bioengineering and therapeutic fields.

In this study, we disclosed TdRNAP as a novel and universal platform for regulating gene expression in response to a wide variety of intracellular molecules. In the examples, it was demonstrated that the variable domain of a single antibody can induce split T7 RNAP assembly upon binding to its corresponding target. Using various identified antibodies against proteins/peptides, RNA, and small molecules, we also demonstrated that TdRNAP can expand its molecular repertoire for inducing gene expression. To our knowledge, this is the first demonstration of a single platform capable of transmitting these three distinct types of biochemical information into transcriptional and translational outputs. In particular, our IRES RNA-dRNAP represents the first example of transmitting RNA structural information into RNA synthesis. Furthermore, by applying TdRNAP, multilayer genetic circuits for signal amplification and orthogonal signal transduction were constructed. Finally, cell-type-specific genome editing was demonstrated, in which TdRNAP autonomously induced gene knockout by detecting intracellular gene products derived from target genes in the human genome.

The present method also demonstrated the advantage that numerous identified antibodies can be used to broaden the target molecules for gene regulation. Because antibody-screening methods are well established and machine-learning-based antibody-design techniques are advancing, antibodies with new molecular specificities can be readily identified and applied to the system of the present invention. Furthermore, the use of the VL and VH domains of antibodies eliminates the need to select split sites, as required in conventional split-protein approaches, thereby enabling facile targeting of diverse intracellular molecules and allowing gene regulation in accordance with cellular states. This advantage greatly broadens the applicability of this molecule-induced gene regulation system.

## Claims

1. A target molecule-specific functional protein regulation system comprising:
the following (a) and (b):
(a) a first nucleic acid molecule including a nucleic acid sequence encoding a first fusion protein that includes a first target binding domain including a light-chain variable domain of an antibody that specifically recognizes a target molecule, and a first domain of a functional protein; and
(b) a second nucleic acid molecule including a nucleic acid sequence encoding a second fusion protein that includes a second target binding domain including a heavy-chain variable domain of an antibody that specifically recognizes the target molecule, and a second domain of a functional protein; or
the following (c) and (d):
(c) the first fusion protein; and
(d) the second fusion protein,
wherein
the first domain of the functional protein is either an N-terminal domain or a C-terminal domain of the functional protein, and the second domain of the functional protein is the other of the N-terminal domain or the C-terminal domain of the functional protein,
a framework region of either the light-chain variable domain or the heavy-chain variable domain includes a sequence of a framework region of trastuzumab or a sequence of a framework region of a mutated sequence thereof, and
the target molecule, the first target binding domain, and the second target binding domain form a ternary complex.

2. The system according to claim 1, wherein the functional protein includes a detection protein, a protein that regulates transcription and translation of RNA, an extracellular secretory protein, a genome-editing protein, an enzyme, and a therapeutic protein.

3. The system according to claim 1, further comprising (e) a third nucleic acid molecule or additional protein regulated by the functional protein.

4. The system according to claim 3, wherein the functional protein is a nucleic acid polymerase, and the third nucleic acid molecule includes a promoter sequence specific for the nucleic acid polymerase and a nucleic acid sequence encoding a protein of interest or an RNA of interest.

5. The system according to claim 4, wherein the protein of interest includes a detection protein, a protein that regulates transcription or translation of RNA, an extracellular secretory protein, a genome-editing protein, an enzyme, and a therapeutic protein.

6. The system according to claim 3, wherein the functional protein is a nucleic acid polymerase, and the third nucleic acid molecule includes a promoter sequence specific for the nucleic acid polymerase and a nucleic acid sequence encoding a guide RNA that specifically recognizes a genome-editing target molecule.

7. The system according to claim 1, wherein the first or second nucleic acid molecule is a DNA construct or a synthetic mRNA molecule.

8. A method for regulating a functional protein in a target molecule-specific manner within a cell, the method comprising a step of introducing the system according to claim 1 into a cell.

9. The method according to claim 8, further comprising a step of introducing (e) a third nucleic acid molecule or additional protein regulated by the functional protein into the cell.
